# EUROPEAN PATENT APPLICATION

(11) **EP 4 260 839 A1**
(43) Date of publication of application: **18.10.2023**
(21) Application number: 21903468.3
(22) Date of filing: 09.12.2021
(51) Int. Cl.: A61J 3/00, B65G 47/14

(54) **DRUG FEEDER**

(30) Priority: 11.12.2020 JP 2020205592; 24.12.2020 JP 2020214571; 07.01.2021 JP 2021001647
(71) Applicant: Tosho, Inc., Tokyo 144-0033 (JP)
(72) Inventor: OMURA, Yoshihito, Tokyo 1440033 (JP); OHGAYA, Syunji, Tokyo 1440033 (JP)
(74) Representative: Wilson Gunn
(86) International application number: PCT/JP2021/045336
(87) International publication number: WO 2022/124369

(57) **Abstract**

The present invention provides a medicine feeder capable of moderately recovering the height regulation function of a tablet height regulation mechanism in such a range that even fragile medicine pieces are not damaged and by a simple means. An alignment regulation mechanism includes a tablet height regulation mechanism 1600 including a plurality of suspended objects suspended over an annular upper end surface, and the tablet height regulation mechanism is configured to regulate heights of medicine pieces using the plurality of suspended objects. The plurality of suspended objects include at least one deformable suspended object 1612, 1632 that is deformed when a lower end portion of the deformable suspended object is pushed sideways and at least one non-deformable suspended object 1623 that is not deformed when a lower end portion of the non-deformable suspended object is pushed sideways with an upper end portion of the non-deformable suspended object being swingably supported.

## Description

### TECHNICAL FIELD

The present invention relates to a medicine feeder operable to automatically feed solid granular medicine pieces such as tablets and ampules in order to automate medicine dispensation performed in hospitals, pharmacies, etc.

### BACKGROUND ART

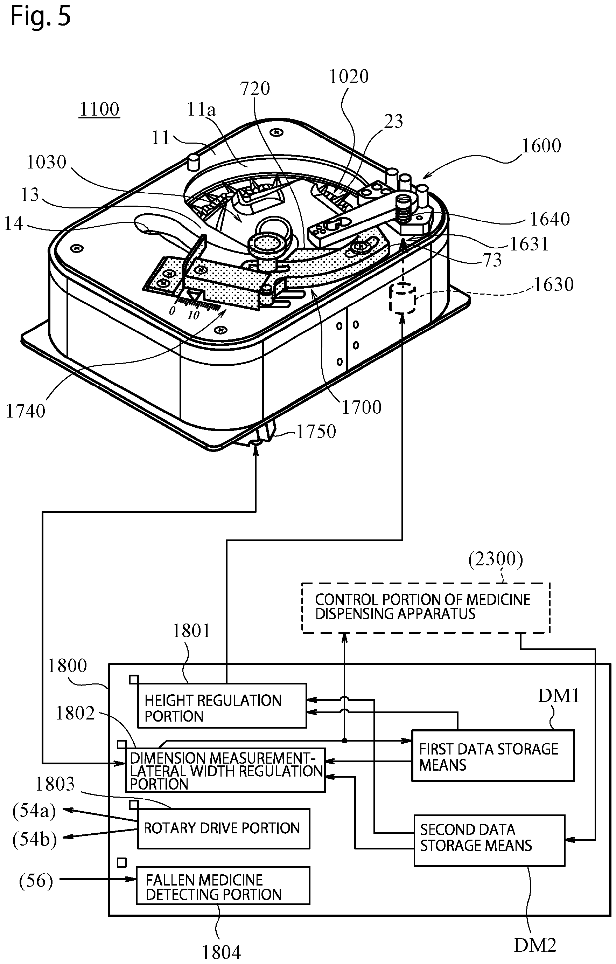

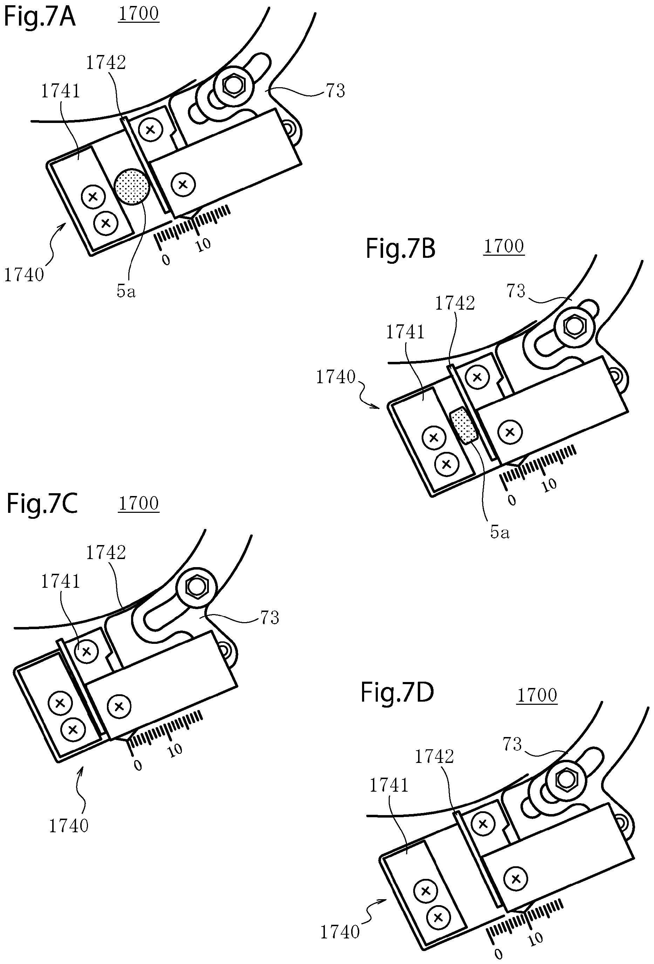

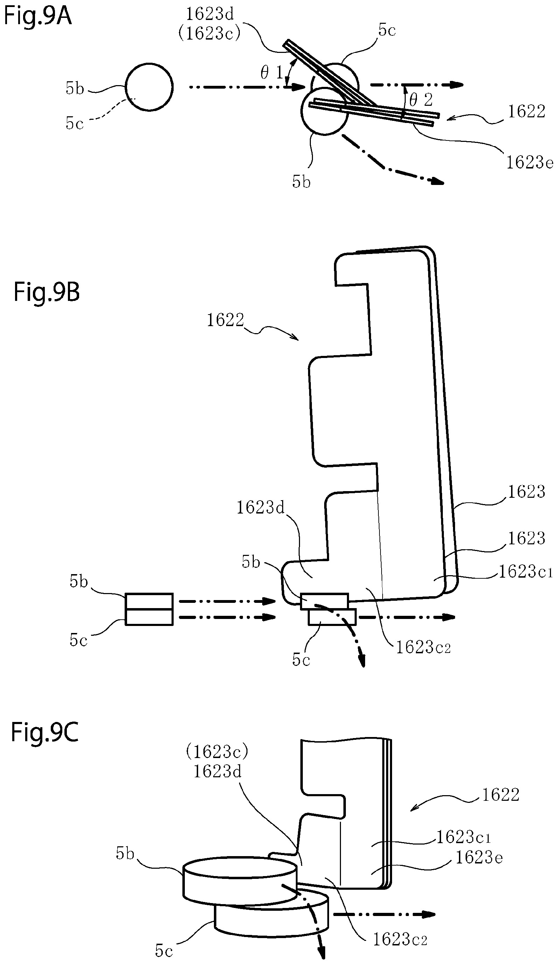

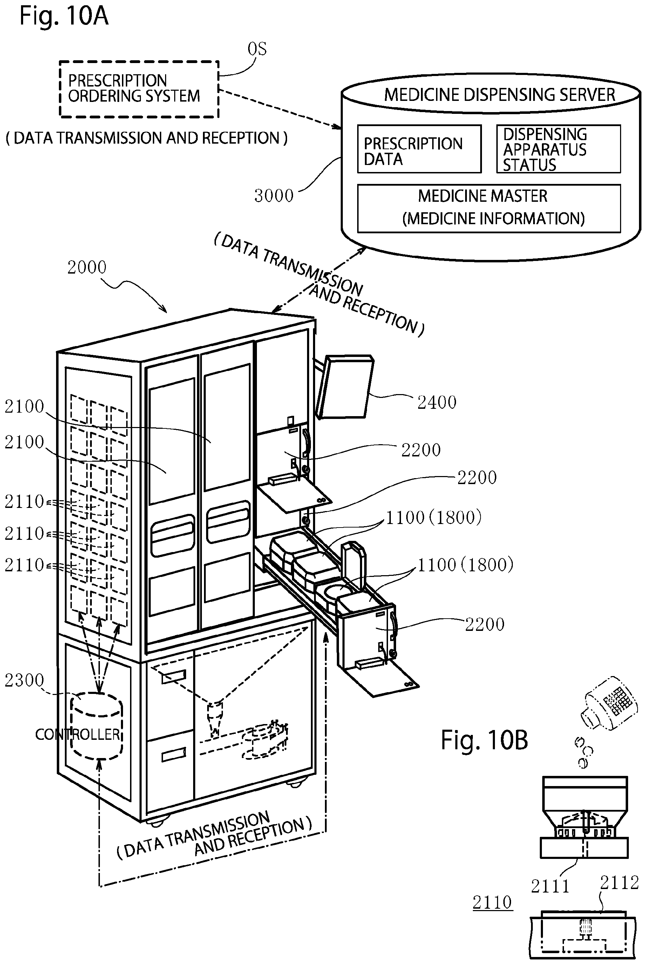

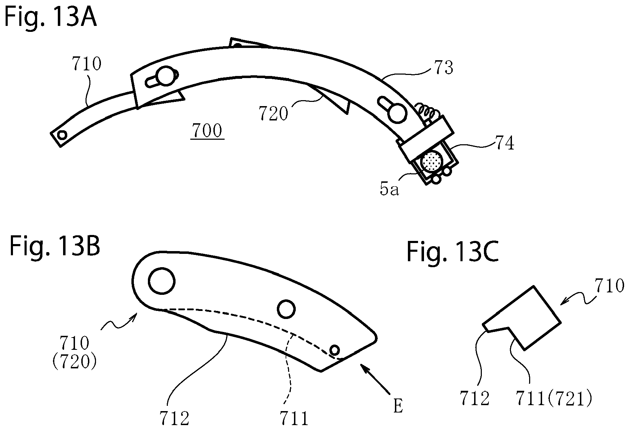

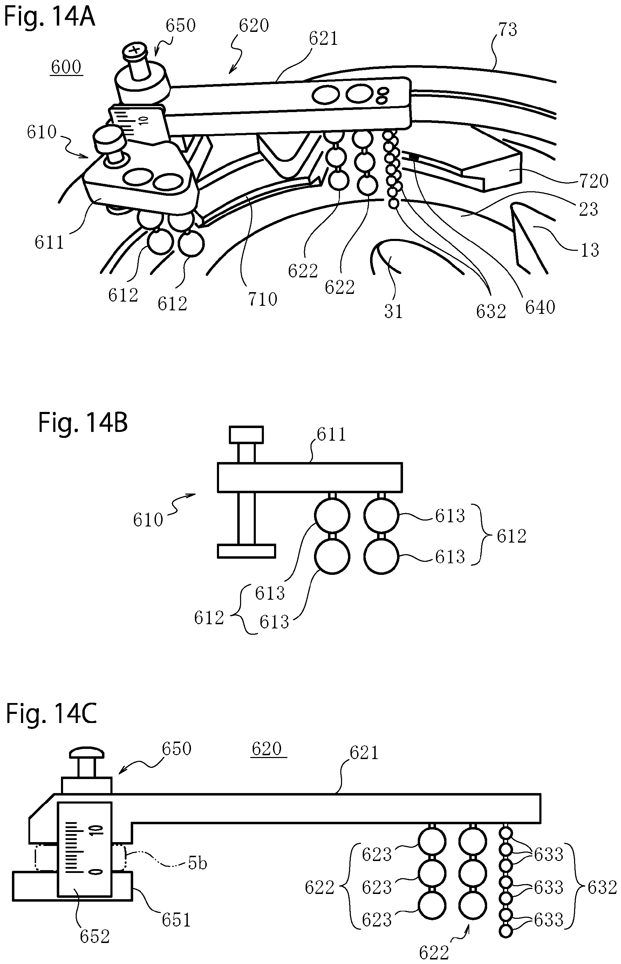

A tablet feeder 10 according to the related art illustrated in Figs. 13 and 14 of the present application is disclosed in Figs. 5, 7, 10, and 9 of Japanese Patent No. 6736075 (JP 6736075; Patent Document 1). Fig. 13 of the present application corresponds to Fig. 9 of the publication, Fig. 14 of the present application corresponds to Fig. 10 of the publication, Fig. 13 of the present application corresponds to a part of Fig. 5 of the publication, and Fig. 14 of the present application corresponds to Fig. 7 of the publication. Thus, the same reference numerals as the numerals given in the publication are given in Figs. 13 and 14 of the present application.

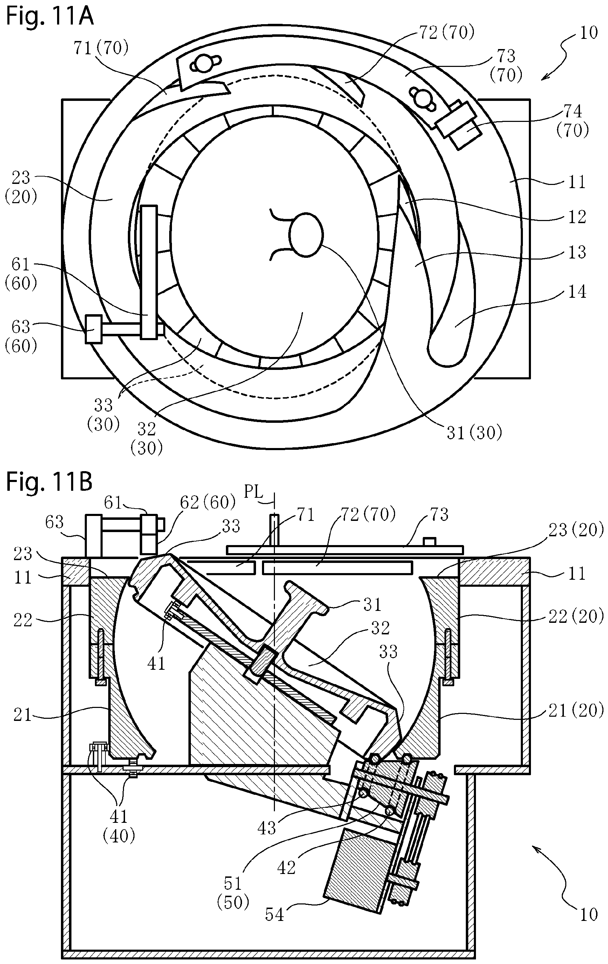

A first tablet feeder 10 according to the related art includes: an outer rotary body 22 including an internal space having an opening portion opening upward and an annular upper end surface 23 surrounding the opening portion, the outer rotary body 20 being rotatable about a virtual vertical line PL extending in a vertical direction in the internal space; and an inner inclined rotary body 30 disposed in the internal space of the outer rotary body 20, the inner inclined rotary body 30 being rotatable about a virtual inclined line tilted with respect to the vertical line PL, with a plurality of solid medicine pieces being placed on an upper surface portion of the inner inclined rotary body 30, to move the plurality of medicine pieces onto the annular upper end surface 23 of the outer rotary body 20 while rotating. The tablet feeder 10 also includes an alignment regulation mechanism 70 configured to align the plurality of medicine pieces, which have been moved onto the annular upper end surface 23 of the outer rotary body 22, in a rotational direction of the annular upper end surface 23 when the outer rotary body 20 is rotating. Portions that help understand the constitution and the issue of the present invention will be specifically described with reference to the drawing (see Fig. 11). Fig. 11 illustrates substantially the entirety of the medicine feeder 10, in which Fig. 11A is a plan view and Fig. 11B is a vertical sectional view.

The medicine feeder 10 in Fig. 11 includes, in addition to the outer rotary body 20 and the inner inclined rotary body 30 discussed earlier, a support mechanism 40 configured to rotatably support the inner inclined rotary body 30 and the outer rotary body 20, a rotary drive mechanism 50 operable to drive rotation of the inner inclined rotary body 30 and the outer rotary body 20, and a tablet height regulation mechanism 60 and a lateral width regulation mechanism 70 provided on the upper side of a peripheral wall 11. The support mechanism 40 keeps the outer rotary body 20 axially rotatable about the plumb line, and keeps the inner inclined rotary body 30 axially rotatable about an inclined line tilted from the plumb line.

The support mechanism 40 is composed of a plurality of members 41 to 43 distributed at various locations. The members 41 to 43 include several passive members 41 mainly composed of radial bearings, for example, and rotation transmission members 42 and 43 composed of a ring-shaped body or an annular body such as an O-ring made of hard rubber, for example. The rotary drive mechanism 50 includes a rotary drive member 51 disposed under the rotary containers 20 and 30 and a rotary drive motor 54 operable to axially rotate the rotary drive member 51, and is operable to axially rotate the outer rotary body 20 at a relatively high speed and axially rotate the inner inclined rotary body 30 at a relatively low speed through friction gearing via the rotation transmission members 42 and 43 with different diameters.

The tablet height regulation mechanism 60 is mainly composed of an elongated bar material extending from a base end portion 61 at the swing fulcrum to a distal end portion 62 at the swing end. The base end portion 61 is supported above the peripheral wall 11 or the outer rotary body 20 by a support portion 63 to allow the distal end portion 62 to be swung up and down about the base end portion 61. A portion that is close to the distal end portion 62 is slightly bent so that the distal end portion 62 is normally lightly placed on a peripheral edge portion 33 of the inner inclined rotary body 30 due to its own weight with the distal end portion 62 being directed obliquely downward. Thus, many of the medicine pieces that have been carried along while being placed on the peripheral edge portion 33, without sliding down from the peripheral edge portion 33 onto the annular upper end surface 23 of the outer rotary body 20, abut against the distal end portion 62 to be returned to a center portion 32 of the inner inclined rotary body 30 by a repulsion biasing force. When such operation is not smoothly performed, the distal end portion 62 may be swung upward in order to avoid damage etc. being given to the medicine pieces.

The lateral width regulation mechanism 70 includes a first regulation member 71 installed ahead of the tablet height regulation mechanism 60 with reference to the rotational direction of the annular upper end surface 23 of the outer rotary body 20, a second regulation member 72 installed further ahead of the first regulation member 71, a link mechanism 73 coupled to each of the first regulation member 71 and the second regulation member 72 via a pin-like rotation allowing shaft member etc., and a sample tablet placement portion 74 at which a sample medicine piece may be accommodated. Swing center portions of the first regulation member 71 and the second regulation member 72 are located on the peripheral wall 11 side, and swing end portions of the first regulation member 71 and the second regulation member 72 are located on the annular upper end surface 23 of the outer rotary body 20, which allows the width of a medicine transfer path on the annular upper end surface 23 to be narrowed from the outer peripheral side.

Moreover, both the first regulation member 71 and the second regulation member 72 may be swung to variably adjust the amount by which the width of the medicine transfer path on the annular upper end surface 23 is narrowed. The regulation members 71 and 72 adjust the amount by which the width of the medicine transfer path is narrowed in conjunction with each other, since the regulation members 71 and 72 are swung at the same time and in the same manner. Further, when a sample medicine piece is accommodated at the sample tablet placement portion 74 and then the link mechanism 73 is moved toward the sample medicine piece, the link mechanism 73 is advanced in the longitudinal direction and stopped with the distal end of the link mechanism 73 abutting against the sample medicine piece. At that time, both the swing end portion of the first regulation member 71 and the swing end portion of the second regulation member 72 narrow the width of the medicine transfer path on the annular upper end surface 23 to a width corresponding to one sample medicine piece.

A fall-discharge port 14 penetrating vertically is formed in the peripheral wall 11 further ahead of the regulation mechanism 70. A discharge guide 13 is also provided in the peripheral wall 11 in order to feed medicine pieces on the annular upper end surface 23 of the outer rotary body 20 to the fall-discharge port 14 through rotation of the outer rotary body 20. A transfer surface guide 12 is formed at the distal end portion of the discharge guide 13 to extend downward and forward therefrom to constitute the foremost end. The transfer surface guide 12 prevents medicine pieces from undesirably falling toward the inner inclined rotary body 30 due to the repulsion of abutment against the discharge guide 13.

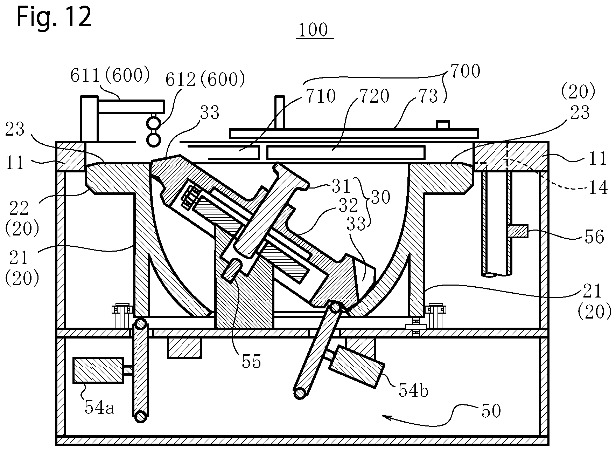

Fig. 12 is a vertical sectional view illustrating the overall configuration of an improved medicine feeder 100 according to the related art. Fig. 13 illustrates the configuration of a new lateral width regulation mechanism 700 obtained by improving the lateral width regulation mechanism 70 in Fig. 13, in which Fig. 13A is a plan view of the lateral width regulation mechanism 700 with a model medicine piece 5a being placed at the sample tablet placement portion 74 and Fig. 13B includes a plan view and an end surface view of a first regulation member 710. Fig. 14 illustrates the configuration of a tablet height regulation mechanism 600 obtained by improving the tablet height regulation mechanism 60 illustrated in Fig. 10 in which Fig. 14A is a perspective view illustrating the appearance of the tablet height regulation mechanism 600 and a portion at which the tablet height regulation mechanism 600 is installed, Fig. 14B is a front view of a first regulation member 610 of the tablet height regulation mechanism 600, and Fig. 14C is a front view of a second regulation member 620 of the tablet height regulation mechanism 600.

The improved medicine feeder 100 in Fig. 12 is different from the medicine feeder 10 before being improved illustrated in Fig. 13 in that the rotary drive mechanism 50 includes two rotary drive motors 54a and 54b to be able to individually control rotation of the outer rotary body 20 and rotation of the inner inclined rotary body 30, that the outer rotary body 20 is a bowl-shaped integral object that allows insertion and extraction of the inner inclined rotary body 30 without dividing the outer rotary body 20 into upper and lower pieces, that inner inclined rotary body mount detecting means 55 for detecting mounting and unmounting of the inner inclined rotary body 30 has been added, and that fallen medicine detecting means 56 under the fall-discharge port 14 is clearly indicated, etc.

The lateral width regulation mechanism 700 includes a sample tablet placement portion 74 for setting a model medicine piece 5a and a link mechanism 73 configured to move the first and second regulation members 71 and 72 in conjunction with each other, which are similar to those of the lateral width regulation mechanism 70 of the first tablet feeder 10 illustrated in Figs. 11A and 11B. The first regulation member 71 and the second regulation member 72 in Fig. 11 are improved into a first regulation member 710 and a second regulation member 720, respectively, in Figs. 13A to 13C. The first regulation member 710 and the second regulation member 720 are of the same shape, and thus the first regulation member 710 is discussed in detail. The first regulation member 710 exhibits a lateral width regulation function to narrow the width of a medicine transfer path on the annular upper end surface 23 of the outer rotary body 20 from the outer peripheral side according to advancement and retraction of the link mechanism 73 in the longitudinal direction, with a swing center portion at the left end in Fig. 13A being located on the peripheral wall 11 side in Fig. 12 and with a swing end portion at the right end being positioned above the annular upper end surface 23.

Moreover, the first regulation member 710 (see Fig. 13B) includes not only a lower portion 711 that exerts the lateral width regulation function, but also an upper portion 712, formed on the inner peripheral side surface. The upper portion 712 overhangs toward the inner peripheral side with respect to the lower portion 711, with an inclined surface being formed between the upper portion 712 and the lower portion 711. Therefore, in the first regulation member 710, the upper portion 712 performs rough height regulation, in addition to the strict lateral width regulation function performed by the lower portion 711 according to the state of the link mechanism 73. The second regulation member 720 is configured in the same manner.

The tablet height regulation mechanism 600 illustrated in Fig. 14 includes a first regulation member 610 disposed upstream of the lateral width regulation mechanism 700 in the medicine transfer path on the annular upper end surface 23 of the outer rotary body 20, and a second regulation member 620 disposed side by side with the lateral width regulation mechanism 700. Each of the first and second regulation members 610 and 620 is mounted to a plate body that includes a discharge guide 13 and that supports the lateral width regulation mechanism 700, as with the regulation member 62 of the tablet height regulation mechanism 60 in Fig. 11B.

The first regulation member 610 (see Figs. 14A and 14B) includes a short support member 611, the vertical position of which is adjustable using a manual screw mechanism, and front suspended objects 612 (suspended objects of a first form) attached to a portion of the support member 611 close to the distal end to be moved vertically together with the support member 611. The front suspended objects 612 (see Fig. 14B) are in a chain shape in which a plurality of large balls 613 (two spherical bodies in the drawing) are loosely coupled to each other. In the first regulation member 610, the two front suspended objects 612 are suspended side by side from the support member 611 over the medicine transfer path on the annular upper end surface 230, and the vertical position of the front suspended objects 612 is adjusted such that the lower ends of the front suspended objects 612 are normally slightly higher than the height of medicine pieces.

The second regulation member 620 (see Figs. 14A and 14C) includes a long support member 621, the vertical position of which is also manually adjustable, middle suspended objects 622 and rear suspended objects 632 (all suspended objects of the first form) attached to a portion of the support member 621 close to the distal end to be moved vertically together with the support member 621, and a manual adjustment mechanism 650 that allows adjustment of the vertical position of the support member 621. The middle suspended objects 622 (see Fig. 14C) are in a chain shape in which a plurality of medium balls 623 (three spherical bodies in the drawing) are loosely coupled to each other, with the number of coupled balls being increased as the medium balls 623 are smaller. The rear suspended objects 632 are also in a chain shape in which a plurality of (seven on the front side and six on the back side in the drawing) small balls 633 are loosely coupled to each other, with the number of coupled balls being increased as the small balls 633 are smaller.

The manual adjustment mechanism 650 sets the vertical position of the support member 621 to a position corresponding to the model medicine piece 5b, by raising the support member 621 through a manual operation to widen the gap between the support member 621 and a lower limit setting mechanism 651, placing a model medicine piece 5b (a medicine piece that is separate from but is in the same shape as the model medicine piece 5a in Fig. 13A placed at the sample tablet placement portion 74 discussed above) on the lower limit setting mechanism 651, and then lowering the support member 621 through a manual operation until the support member 621 lightly contacts the model medicine piece 5b. The lower limit setting mechanism 651 is provided with a scale member 652 (see Fig. 14C).

Patent Document 1: Japanese Patent No. 6736075

### SUMMARY OF INVENTION

### TECHNICAL PROBLEM

When a medicine feeder according to the related art is used for a long time for a variety of medicine pieces of different shapes and materials, the following situation occurs although on rare occasions. That is, medicine pieces in a so-called stacked state, in which a plurality of medicine pieces are vertically stacked on each other, occasionally escape from height regulation by the tablet height regulation mechanism in the former stage to reach the lateral width regulation mechanism in the latter stage. In this state, stacked medicine pieces are not disentangled by the height regulation function of the lateral width regulation mechanism and it takes a long time for the medicine pieces to fall from the medicine transfer path on the outer rotary body onto the inner inclined rotary body on the inner side by the width regulation function of the lateral width regulation mechanism. This may trigger a large number of subsequent medicine pieces to continuously stay on the medicine transfer path on the outer rotary body.

When such a situation that may well be called a congested state occurs, the frequency of transfer of medicine pieces from the lateral width regulation mechanism to the fall-discharge port is decreased, and thus the function to sequentially discharge medicine pieces is impaired beyond expectation. As a result, the processing capability may be reduced to an undesirable degree and, further, an undesirable time-out may occur during medicine discharge detection, thereby making an erroneous judgment that all the medicine pieces in the feeder are completely discharged and the feeder has become empty. Thus, it is a problem to be solved to moderately recover the height regulation function of the tablet height regulation mechanism in such a range that even fragile medicine pieces are not damaged and by a simple means.

### SOLUTION TO PROBLEM

The present invention provides a medicine feeder including: an outer rotary body including an internal space having an opening portion opening upward and an annular upper end surface surrounding the opening portion, the outer rotary body being rotatable about a virtual vertical line extending in a vertical direction in the internal space; an inner inclined rotary body disposed in the internal space of the outer rotary body, the inner inclined rotary body being rotatable about a virtual inclined line tilted with respect to the vertical line, with a plurality of solid medicine pieces being placed on an upper surface portion of the inner inclined rotary body, to move the plurality of medicine pieces onto the annular upper end surface of the outer rotary body while rotating; and an alignment regulation mechanism configured to align the plurality of medicine pieces, which have been moved onto the annular upper end surface of the outer rotary body, in a rotational direction of the annular upper end surface when the outer rotary body is rotating. The above virtual vertical line is virtual, and is typically a plumb line discussed earlier. However, the virtual vertical line may be tilted from the plumb line to such a small degree that the medicine transfer function of the outer rotary body is not impaired.

The alignment regulation mechanism includes a tablet height regulation mechanism including a plurality of suspended objects suspended over the annular upper end surface, the tablet height regulation mechanism being configured to regulate heights of the medicine pieces using the plurality of suspended objects. The plurality of suspended objects include at least one deformable suspended object that is deformed when a lower end portion of the deformable suspended object is pushed sideways and at least one non-deformable suspended object that is not deformed when a lower end portion of the non-deformable suspended object is pushed sideways with an upper end portion of the non-deformable suspended object being swingably supported.

With the medicine feeder according to the present invention, not only deformable suspended objects that are deformable when the lower end portion is pushed sideways but also non-deformable suspended objects that are not deformed but are swingable when the lower end portion is pushed sideways with the upper end portion being loosely supported are provided and used together as a plurality of suspended objects for moderately resolving stacked medicine pieces . Each of the suspended objects generates a repulsion force to disentangle stacked medicine pieces transferred by the outer rotary body when the stacked medicine pieces abut against the suspended object. How a repulsion force is generated and the generated repulsion force acts is different between when the suspended objects are deformed and when the suspended objects are swung. Thus, a good effect of resolving stacked medicine pieces can be expected compared to when a repulsion force of the same nature repeatedly acts. Since the end portions of the suspended objects abutting against stacked medicine pieces are swung sideways, an excessive repulsion force is not likely to occur compared to when the end portions of the suspended objects are moved upward. Thus, with the present invention, it is possible to moderately recover the height regulation function of the tablet height regulation mechanism in such a range that even fragile medicine pieces are not damaged and by a simple means.

In the medicine feeder according to the present invention, the at least one deformable suspended object and the at least one non-deformable suspended object may be provided at different positions as seen in a circumferential direction of the outer rotary body. Preferably, the plurality of suspended obj0ects are arranged in the circumferential direction in order of the at least one deformable suspended object, the at least one non-deformable suspended object, and at least one different deformable suspended object. With such a configuration, the plurality of suspended objects can be readily disposed on the medicine transfer path. The plurality of suspended objects of the plurality of different types have different shapes and structures, and therefore apply different impacts to a plurality of stacked tablets when the suspended objects hit the plurality of tablets. In particular, when the plurality of suspended objects of the plurality of different types are disposed at different positions as seen in the circumferential direction, different "actions to resolve a stacked state (hereinafter referred to as "actions to disentangle stacked medicine pieces")" can be given to stacked tablets at time intervals from the plurality of suspended objects of the plurality of different types. Thus, it is possible to conveniently avoid the occurrence of an undesired event in which a plurality of "actions to disentangle stacked medicine pieces" cancel out each other.

Preferably, the at least one deformable suspended object preferably has a structure that is adapted to allow the lower end portion of the deformable suspended object to be deformed along an outer peripheral surface of the tablets and generate a repulsion force to disentangle the plurality of stacked tablets when a plurality of stacked tablets located on the annular upper end surface abut against the at least one deformable suspended object. Preferably, the at least one non-deformable suspended object has a structure that is adapted to generate a repulsion force to disentangle the plurality of stacked tablets and move the tablets toward the opening portion of the outer rotary body along the lower end portion of the non-deformable suspended object when a plurality of stacked tablets located on the annular upper end surface abut against the at least one non-deformable suspended object.

More specifically, the tablet height regulation mechanism has a structure that includes at least one support arm configured to support respective upper end portions of the plurality of suspended objects, and a through hole penetrating in the vertical direction formed in the support arm to be penetrated by the upper end portion of the non-deformable suspended object. In this case, a projecting portion projecting in the same direction as the upper end portion is provided between the upper end portion and the lower end portion of the non-deformable suspended object. Preferably, a slit portion is provided under the through hole, with the projecting portion being loosely fitted in the slit portion, to restrict motion of the projecting portion such that the non-deformable suspended object is swung in a limited range when the stacked tablets abut against the lower end portion. With such a configuration, the action to disentangle stacked medicine pieces can be enhanced by causing variations in the repulsion force given to stacked tablets with the lower end portion being slightly swung when the tablets abut against the lower end portion.

Preferably, an entire portion of the non-deformable suspended object or a portion of the non-deformable suspended object including the lower end portion is in a plate shape, and the lower end portion includes a first portion located in a first virtual plane together with the projecting portion and a second portion that is continuous with the first portion and located in a second virtual plane intersecting the first virtual plane at a predetermined angle θ. In this case, the slit portion positions the non-deformable suspended object such that the first portion extends along a rotational direction of the outer rotary body and the second portion is located on a rear side in the rotational direction with respect to the first portion and extends outward in a radial direction of the outer rotary body. With such a configuration, the lower end portion of the non-deformable suspended object is inclined with respect to the moving direction (medicine transfer direction) of the annular upper end surface of the outer rotary body. The inclination of the lower end portion is varied by swing of the non-deformable suspended object. Since the range of the swing is limited, the transfer direction of a medicine piece transferred in the state of being placed on another medicine piece can be smoothly changed to the inner peripheral side of the outer rotary body through abutment with the non-deformable suspended object. As a result, the action to disentangle stacked medicine pieces and the action to return extra medicine pieces into the internal space of the outer rotary body can be enhanced, even with a simple configuration, by installing the non-deformable suspended object.

When the non-deformable suspended object is constituted by arranging a plurality of separable members side by side, the plurality of members may be slightly displaced from each other, or be finely separated from and brought into contact with each other, when abutting against medicine pieces. Thus, the peak of repulsion forces for the medicine pieces from the plurality of members tends to be lowered, even when the total amount of the repulsion forces for the medicine pieces is not changed. As a result, with this configuration, it is possible to enhance the action to disentangle stacked medicine pieces while suppressing an undesirable impact etc. on the medicine pieces.

When the plurality of members are all plate materials of the same shape, the burden of manufacturing the non-deformable suspended object is reduced. When the non-deformable suspended object is constituted by arranging a plurality of plate materials of the same shape side by side, in addition, it is easy to dispose the plurality of plate materials in close contact with each other (in a dense arrangement).

The medicine feeder according to the present invention may further include a control portion configured to control rotation of the outer rotary body and fallen medicine detecting means for detecting a fallen medicine piece that has been carried to a fall-discharge port by the outer rotary body. The alignment regulation mechanism includes a lateral width regulation mechanism configured to regulate a lateral width of a medicine transfer path on the annular upper end surface of the outer rotary body. The lateral width regulation mechanism is configured to adjust a regulation amount of the lateral width according to a control instruction from the control portion. The lateral width regulation mechanism increases the lateral width according to the control instruction from the control portion when an interval of detection for fallen medicine pieces by the fallen medicine detecting means reaches a congestion-time interval which is longer than a normal-time interval. The above interval of detection for fallen medicine pieces includes both a time interval since the start of rotation of the outer rotary body until the detection of a medicine piece by the fallen medicine detecting means and a time interval since the detection of the preceding medicine piece until the detection of the following medicine piece by the fallen medicine detecting means.

When the fallen medicine detecting means detects a fallen medicine piece after the lateral width regulation mechanism increases the lateral width according to the control instruction from the control portion, the lateral width regulation mechanism may restore the lateral width to the state before being increased according to a control instruction from the control portion provided according to such detection of the fallen medicine piece.

The medicine detection interval for medicine pieces carried to the fall-discharge port by the outer rotary body and having fallen is equal to or close to the known normal-time interval (time interval during normal times) under normal operation to discharge medicine pieces. However, when the medicine detection interval reaches a congestion-time interval (time interval during congestion times) that is longer than the time interval during normal times, there is a high possibility that medicine pieces are in an undesirable congested state in the medicine transfer path on the annular upper end surface of the outer rotary body. Thus, when the lateral width regulation mechanism is actuated to increase the lateral width of the medicine transfer path, regulation on the passage of medicine pieces beside the lateral width regulation mechanism is temporarily mitigated. As a result, when the lateral width regulation mechanism is clogged with a large number of medicine pieces and the medicine pieces which are continuous in the transfer direction are in a congested state, such a state is detected to allow the medicine piece at the head of the congestion to easily advance or go forward. Then, slight motion of the medicine piece at the head mitigates the push-and-shove action (pushing each other) of the following medicine pieces to cause a change in a queue of the medicine pieces, which helps resolve the congested state of the medicine pieces . Moreover, by causing the lateral width regulation mechanism to slightly operate, the congested state of the medicine pieces can be conveniently mitigated, and undesirable push-and-shove (pushing each other) of the medicine pieces is mitigated. Thus, there is no fear that the medicine pieces are damaged even if the medicine pieces are fragile. Thus, in this manner, it is possible to resolve congestion of the medicine pieces by exhibiting the height regulation function of the lateral width regulation mechanism in such a range that even fragile medicine pieces are not damaged and by a simple means.

The lateral width regulation mechanism may increase the lateral width stepwise according to the control instruction from the control portion. When the lateral width of the medicine transfer path is increased stepwise, rather than continuously, medicine pieces to be regulated are intermittently moved in the lateral direction. Thus, friction among the medicine pieces is suppressed and the push-and-shove action (pushing each other) of the medicine pieces is mitigated during the movement. As a result, when the lateral width is increased stepwise, the medicine pieces are aligned during intervals between movements of the medicine pieces in the lateral direction. Thus, the congested state of the medicine pieces can be resolved readily and efficiently by suppressing an impact on the medicine pieces.

The medicine feeder according to the present invention may further include a dimension measurement mechanism operable to clamp a medicine piece and measure a dimension of the medicine piece and a dimension measurement drive mechanism configured to move a movable portion of the dimension measurement mechanism. In this case, preferably, the lateral width regulation mechanism is configured to set an initial value of the lateral width in conjunction with the movable portion of the dimension measurement mechanism. When the control portion acquires medicine dimension data on the medicine piece placed on a dimension measurement portion of the dimension measurement mechanism by driving the dimension measurement drive mechanism, the control portion outputs the control instruction to cause the lateral width regulation mechanism to regulate the lateral width based on the acquired medicine dimension data. With such a configuration, the lateral width regulation mechanism and the movable portion of the dimension measurement mechanism are caused to operate in conjunction with each other, and the lateral width regulation mechanism is also actuated when a dimension measurement drive member moves the movable portion. Thus, not only acquisition of dimension data on a medicine piece placed on the dimension measurement mechanism but also temporary mitigation of the lateral width of the medicine transfer path based on such dimension data can be implemented by the control portion controlling the dimension measurement drive member. The medicine piece used for the dimension measurement can be automatically dispensed without being wasted by taking the medicine piece out of the dimension measurement mechanism after the dimension measurement and transferring the medicine piece onto the inner inclined rotary body. Further, the dimension measurement mechanism can be simplified by acquiring or calculating dimension data from the amount of operation of the dimension measurement drive member.

Preferably, the tablet height regulation mechanism includes an elevating mechanism operable to elevate and lower the plurality of suspended objects, and the medicine feeder further includes first data storage means for holding the medicine dimension data acquired by the dimension measurement mechanism, and second data storage means for acquiring and holding medicine dimension data transmitted from an upper-level device. In this case, the lateral width regulation mechanism may be caused to regulate the lateral width and the elevating mechanism of the tablet height regulation mechanism may be caused to regulate the height based on the medicine dimension data held by one of the first data storage means and the second data storage means.

With such a configuration, the lateral width regulation mechanism and the movable portion of the dimension measurement mechanism are caused to operate in conjunction with each other, in addition to the tablet height regulation mechanism elevating and lowering the plurality of suspended objects, and not only the dimension measurement mechanism but also the lateral width regulation mechanism is actuated when the dimension measurement drive member moves the movable portion. Thus, three functions including acquiring dimension data on a medicine piece placed on the dimension measurement mechanism and regulating not only the lateral width of the medicine transfer path but also the height of medicine pieces passing on the medicine transfer path based on such dimension data are implemented by the control portion controlling the two regulation mechanisms. Moreover, the medicine feeder includes the first data storage means for obtaining medicine dimension data using the dimension measurement mechanism and the second data storage means for obtaining medicine dimension data through data transmission and reception from the upper-level device etc. Thus, operation to consecutively discharge medicine pieces can be immediately started by receiving medicine dimension data that are necessary to regulate the lateral width and the height of the medicine transfer path when such data are already held by the upper-level device etc. As a result, it is only necessary to acquire necessary medicine dimension data by performing dimension measurement when the upper-level device etc. does not hold necessary medicine dimension data, which helps improve the overall medicine packing capability. Thus, the burden of work such as dimension measurement and data setting is reduced in this manner.

It is not essential that medicine dimension data obtained through dimension measurement and medicine dimension data obtained through reception should be used at the same time, which allows the first data storage means and the second data storage means to share a data storage memory.

Preferably, when the control portion acquires the medicine dimension data from the first data storage means, the control portion transmits the medicine dimension data to the upper-level device. In this manner, necessary data can be received from the upper-level device when medicine pieces of the same type are subsequently consecutively discharged, which allows operation to consecutively discharge medicine pieces to be immediately started by omitting dimension measurement operation.

The present invention can also be grasped as a medicine dispensing system. The medicine dispensing system includes a medicine dispensing apparatus in which the medicine feeder according to the present invention is mounted in a medicine storage, and a medicine dispensing server configured to hold prescription data based on information on a prescription and a medicine master including a collection of data on various types of medicine. The medicine dispensing server and the medicine dispensing apparatus are able to transmit and receive data, and a control portion of the medicine dispensing apparatus and the control portion of the medicine feeder are able to transmit and receive data. When the medicine dispensing apparatus receives medicine dispensation instruction data including medicine to be processed by the medicine feeder from the medicine dispensing server, the medicine dispensing apparatus checks whether or not the medicine dispensation instruction data include medicine dimension data on the medicine, and transmits the medicine dimension data to the medicine feeder if the medicine dimension data are included, and instructs the medicine feeder to acquire medicine dimension data using the dimension measurement mechanism and transfers the medicine dimension data transmitted from the medicine feeder to the medicine dispensing server if not.

In this manner, specifically when the medicine dispensing server already holds medicine dimension data on medicine pieces to be processed by the medicine feeder, a medicine dispensation instruction containing such data is sent from the medicine dispensing server to the medicine dispensing apparatus. In the medicine dispensing apparatus, operation to consecutively discharge medicine pieces can be immediately started after the medicine feeder receives the medicine dimension data. Thus, it is not necessary to perform dimension measurement in advance, and advantageously there is no time loss.

When the medicine dispensing server does not hold medicine dimension data on medicine pieces to be processed by the medicine feeder yet, the medicine dimension data are not provided by the medicine dispensation instruction sent from the medicine dispensing server to the medicine dispensing apparatus. In that case, however, necessary data can be prepared by performing dimension measurement using an improved feeder, which allows executing medicine dispensation. Moreover, the medicine dimension data are sent to the medicine dispensing server, which thereafter allows all the improved feeders under management by the medicine dispensing server to make use of the medicine dimension data.

### BRIEF DESCRIPTION OF DRAWINGS

Fig. 1A is a perspective view illustrating the appearance of a tablet height regulation mechanism of a medicine feeder according to a first embodiment of the present invention and structures around the tablet height regulation mechanism, and Fig. 1B is a perspective view illustrating the appearance of the tablet height regulation mechanism alone.
Figs. 2A to 2E are a plan view, a front view, a left side view, a back view, and a right side view, respectively, of a non-deformable suspended object as a single middle suspended object.
Figs. 3A to 3E are a plan view, a front view, a left side view, a back view, and a right side view, respectively, of a combined middle suspended object obtained by providing two middle suspended objects adjacent to each other.
Figs. 4A to 4C illustrate the middle suspended object in a suspended state.
Fig. 5 illustrates the relationship between a dimension measurement mechanism and a lateral width regulation mechanism.
Figs. 6A and 6B are each a perspective view illustrating the appearance of the dimension measurement mechanism.
Figs. 7A to 7D are each a plan view illustrating a movable clamping portion of the dimension measurement mechanism and a link mechanism of the lateral width regulation mechanism operating in conjunction with each other.
Fig. 8 is a perspective view illustrating the appearance of the tablet height regulation mechanism etc. classifying medicine pieces that have come to the combined middle suspended object.
Figs. 9A and 9B are a plan view and a front view, respectively, illustrating the combined middle suspended object classifying medicine pieces in a swung state, and Fig. 9C illustrates the relationship between the lower end portion of the combined middle suspended object classifying medicine pieces and tablets.
Fig. 10A is a schematic view illustrating the configuration of a medicine dispensing system, and Fig. 10B is a front view of a medicine feeder of a cassette mount-unmount type with a cassette being removed.
Fig. 11A and 11B are a plan view and a vertical sectional front view, respectively, illustrating the structure of an essential portion of a medicine feeder according to the related art.
Fig. 12 is a vertical sectional front view illustrating the structure of an essential portion of the medicine feeder according to the related art.
Fig. 13A is a plan view illustrating the structure of a regulation mechanism of the medicine feeder according to the related art with a model medicine piece being placed at a sample tablet placement portion, and Figs. 13B and 13C are a plan view and an end surface view, respectively, of a first regulation member.
Fig. 14A is a perspective view illustrating the structure of a tablet height regulation mechanism of the medicine feeder according to the related art and a portion at which the tablet height regulation mechanism is installed, Fig. 14B is a front view of the first regulation member, and Fig. 14C is a front view of a second regulation member.

### DESCRIPTION OF EMBODIMENTS

A medicine feeder according to an embodiment of the present invention will be described in detail below with reference to the drawings. In the following description, parts that are the same as those known in the art, described in the Background Art section and illustrated in Figs. 11 to 14, will not be repeatedly described, and differences from the related art will be mainly described below.

### (First Embodiment)

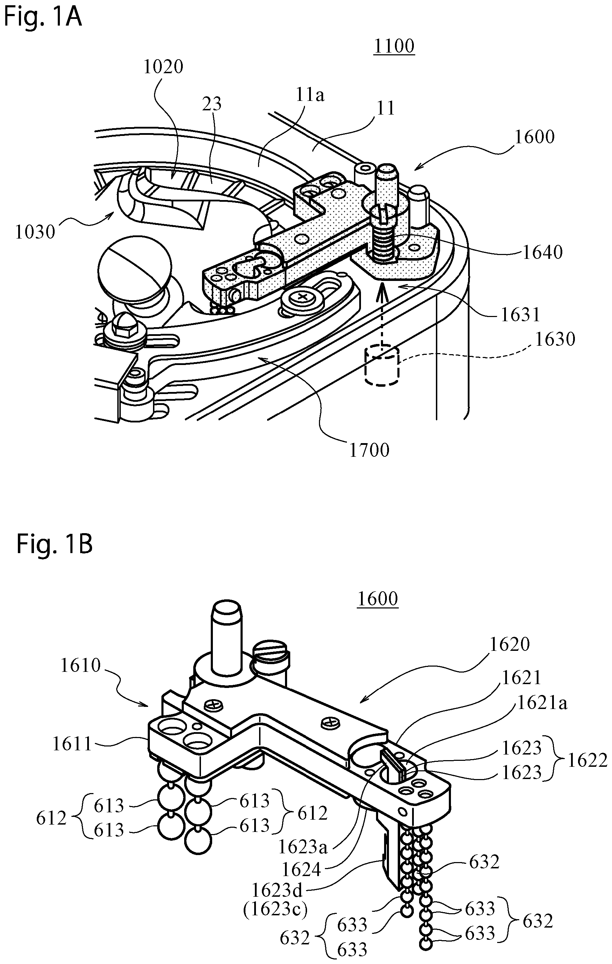

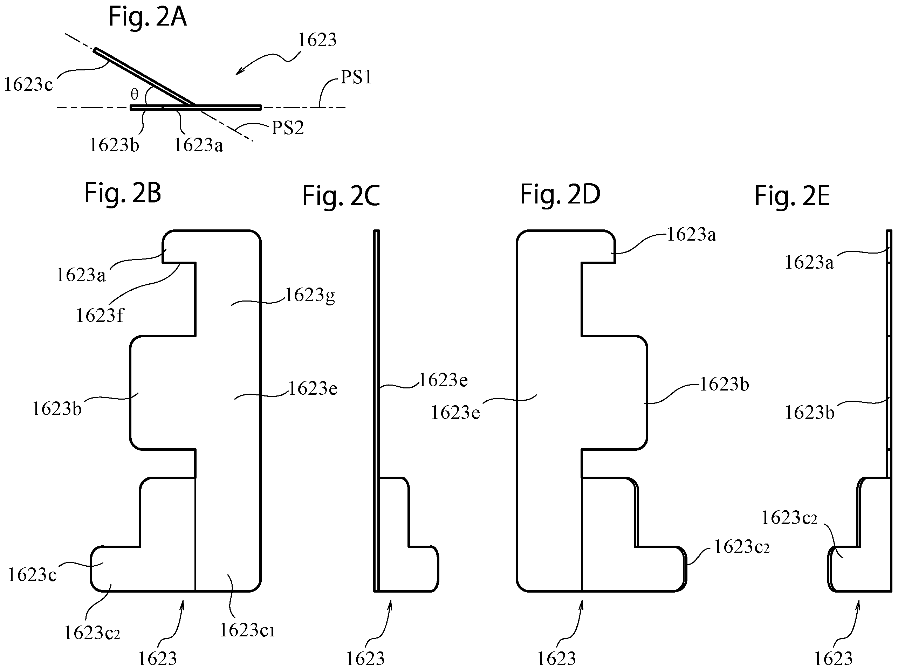

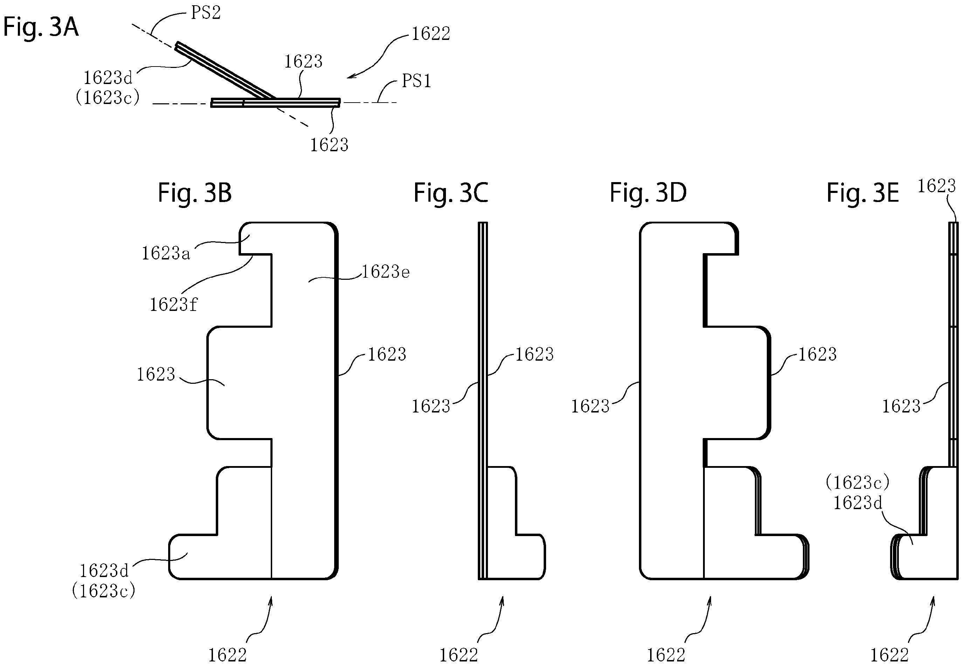

Fig. 1 illustrates the structure of an essential portion of a medicine feeder 1100 according to a first embodiment, in which Fig. 1A is a perspective view illustrating the appearance of a tablet height regulation mechanism 1600 and structures around the tablet height regulation mechanism 1600, and Fig. 1B is a perspective view illustrating the appearance of the tablet height regulation mechanism 1600 alone. Figs. 2A to 2E are a plan view, a front view, a left side view, a back view, and a right side view, respectively, of a non-deformable suspended object as a single middle suspended object 1623. Figs. 3A to 3E are a plan view, a front view, a left side view, a back view, and a right side view, respectively, of a combined middle suspended object 1622 obtained by providing two middle suspended objects (non-deformable suspended objects) 1623 and 1623 adjacent to each other.

The basic configuration of the medicine feeder 1100 illustrated in Fig. 1 is the same as the configuration of the medicine feeders 10 and 100 according to the related art illustrated in Figs. 11 and 12, respectively. Specifically, the medicine feeder 1100 includes a housing peripheral wall 1011 that is similar to the housing peripheral wall 11 known in the art and illustrated in Figs. 11 and 14, an outer rotary body 1020 on the outer side that is rotatable about a virtual vertical line as with the outer rotary body 20 known in the art and illustrated in Figs. 11 and 14, and an inner inclined rotary body 1030 mounted inside the outer rotary body 1020 to occupy a hollow of the outer rotary body 1020, the inner inclined rotary body 1030 being axially rotatable about an inclined line tilted from the virtual vertical line as with the inner inclined rotary body 30 known in the art.

The medicine feeder 1100 (see Fig. 1A) also includes a tablet height regulation mechanism 1600 and a lateral width regulation mechanism 1700 operable to align solid medicine pieces, which have been carried from the top of the inner inclined rotary body 1030 to the top of an annular upper end surface 23 of the outer rotary body 1020 through rotation of the inner inclined rotary body 1030, during rotation of the outer rotary body 1020. The lateral width regulation mechanism 1700 is structured to variably adjust the lateral width of a medicine transfer path through electrical action. The basic structure of the lateral width regulation mechanism 1700 is the same as the structure of the lateral width regulation mechanism 700 known in the art. The lateral width regulation mechanism 1700 regulates the lateral width of a medicine transfer path on the annular upper end surface 23 of the outer rotary body 1020, and regulates the final height of medicine pieces.

### [Tablet Height Regulation Mechanism]

The tablet height regulation mechanism 1600 (see the dotted portion in Fig. 1A and Fig. 1B) loosely, rather than strictly, regulates the height of medicine pieces on the annular upper end surface 23 of the outer rotary body 1020 that can pass through the tablet height regulation mechanism 1600. In the present embodiment, the tablet height regulation mechanism 1600 includes, as three types of suspended objects suspended over the annular upper end surface 23 of the outer rotary body 1020, namely, two front suspended objects 612 known in the art obtained by vertically stringing three large balls 613 to be freely bendable, a combined middle suspended object 1622 constituted by arranging two plate-like middle suspended objects 1623, which will be discussed in detail later, side by side, and three rear suspended objects 632 obtained by vertically stringing a plurality of small balls 633 to be freely bendable. In the present embodiment, the two front suspended objects 612 and the three rear suspended objects 632 constitute at least one deformable suspended object that is deformed when a lower end portion of the deformable suspended object is pushed sideways. The combined middle suspended object 1622 constitutes at least one non-deformable suspended object that is not deformed when a lower end portion of the non-deformable suspended object is pushed sideways with an upper end portion of the non-deformable suspended object being swingably supported. The two front suspended objects 612, the combined middle suspended object 1622, and the three rear suspended objects 632 are disposed at intervals from the upstream side to the downstream side in the rotational direction of the annular upper end surface 23, that is, at intervals in the circumferential direction. The two front suspended objects 612, the combined middle suspended object 1622, and the three rear suspended objects 632 are provided at different positions as seen in the circumferential direction of the outer rotary body 1020. That is, the suspended objects 612, 1622, and 632 are provided at different radial positions as seen in the circumferential direction.

While a support arm 1611 configured to suspend and hold the plurality of front suspended objects 612 is short, a support arm 1621 configured to suspend and hold the combined middle suspended object 1622 and the rear suspended objects 632 is long enough to extend over the lateral width regulation mechanism 1700 . Since the two support arms 1611 and 1621 are fabricated from an integral object or tightly coupled to each other, a first regulation member 1610 including the support arm 1611 and a second regulation member 1620 including the support arm 1621 are also integrated with each other. Therefore, in the present embodiment, the respective lower end positions of the three types of suspended objects 612, 1622, and 632 can be collectively automatically adjusted vertically by an elevating mechanism 1631 moving the first regulation member 1610 and the second regulation member 1620 together in the vertical direction, the elevating mechanism 1631 being vertically movable by an electric motor 1630 as illustrated in Figs. 1 and 5. That is, when the motor 1630 (classification drive member) is actuated, an elevating screw 1640 of the elevating mechanism 1631 is driven to be axially rotated, and accordingly the support arms 1611 and 1621 as an integral object are elevated and lowered.

When the front suspended objects 612 and the rear suspended objects 632 are pushed sideways with a medicine piece transferred by the annular upper end surface 23 of the outer rotary body 1020 colliding against the lower end portions of the front suspended objects 612 and the rear suspended objects 632, the front suspended objects 612 and the rear suspended objects 632 mitigate a shock while holding back the medicine piece having collided. Then, the front suspended objects 612 and the rear suspended objects 632 are arched as a whole so that portions that are closer to the lower end portions are more easily and significantly deformed. The front suspended objects 612 and the rear suspended objects 632, which are deformed in this manner, constitute the at least one deformable suspended object.

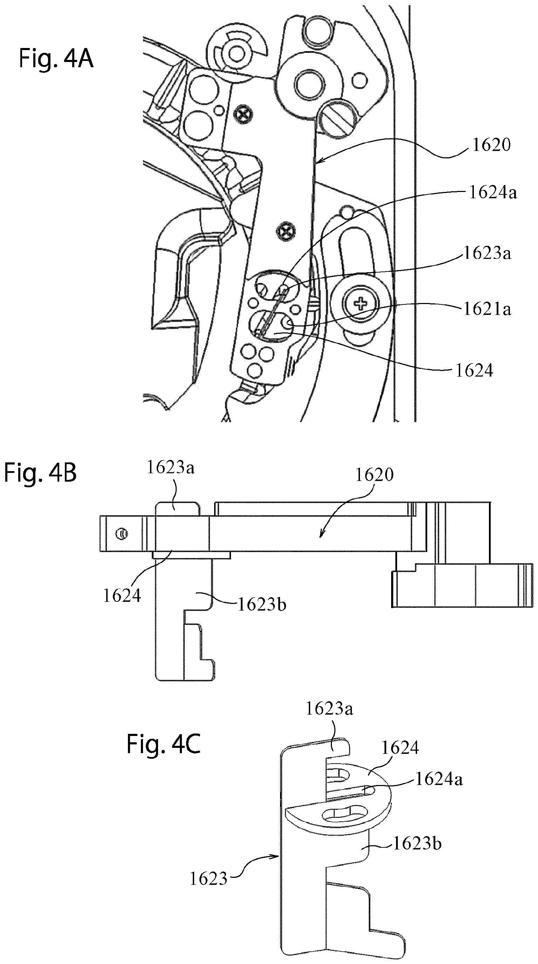

On the contrary, the combined middle suspended object 1622, which is obtained by stacking the middle suspended objects 1623 constituted from thin and vertically long plate-like bodies and not easily deformable, are loosely suspended with upper end portions (1623a) loosely fitted and caught in a through hole 1621a of the support arm 1621. The combined middle suspended object 1622 constitutes a single non-deformable suspended object that is not deformed but is swingable when the lower end portion is pushed sideways. More specifically, the through hole 1621a extending in the vertical direction is formed in the support arm 1621 which swingably supports the upper end portions 1623a of the two middle suspended objects 1623 which constitute the combined middle suspended object 1622. The upper end portions 1623a of the two middle suspended objects 1623 include a penetrating portion 1623g loosely penetrating the through hole 1621a and an engaging portion 1623f that is continuous with the penetrating portion and that engages with the upper surface portion of the support arm 1621 surrounding the upper opening of the through hole 1621a. A projecting portion 1623b projecting in the same direction as the upper end portion 1623a is provided between the upper end portion 1623a and a lower end portion 1623dc of the middle suspended object 1623. As illustrated in Figs. 4A to 4C, a slit portion 1624 including a slit 1624a is fixed under the through hole 1621a. The slit 1624a of the slit portion 1624, in which the projecting portion 1623b is loosely fitted, is shaped to restrict motion of the middle suspended object 1623 having the projecting portion 1623b such that the middle suspended object 1623 is swung in a limited range when stacked tablets abut against the lower end portion 1623c. When such a slit portion 1624 is provided, a repulsion force to be given to tablets is varied with the lower end portion 1623c being slightly swung when the tablets abut against the lower end portion 1623c, thereby enhancing the effect of disentangling stacked medicine pieces.

As illustrated in Fig. 2, the entire portion of the middle suspended object 1623 or a portion of the middle suspended object 1623 including the lower end portion 1623c is in a plate shape, and the lower end portion 1623c includes a first portion 1623c1 located in a first virtual plane PS1 together with the projecting portion 1623b and a second portion 1623c2 that is continuous with the first portion 1623c1 and located in a second virtual plane PS2 intersecting the first virtual plane PS1 at a predetermined angle θ. In this case, the slit portion 1624 is used to position the middle suspended object 1623 such that the first portion 1623c1 extends along the rotational direction of the outer rotary body 1020 and the second portion 1623c2 is located on the rear side in the rotational direction with respect to the first portion 1623c1 and extends outward in the radial direction of the outer rotary body 1020. When the slit portion 1624 is used, the lower end portion 1623c of the middle suspended object 1623 is inclined with respect to the moving direction (medicine transfer direction) of the annular upper end surface of the outer rotary body 1020. The inclination of the lower end portion 1623c is varied by swing of the middle suspended object 1623. The range of the swing is limited by the width of the slit 1624a.

As illustrated in Fig. 3, the combined middle suspended object 1622 which is obtained by arranging two (a plurality of) middle suspended objects 1623 side by side in a close but easily separable state constitutes one or more non-deformable suspended objects arranged side by side. The combined middle suspended object 1622 is deformed differently from the front suspended objects 612 and the rear suspended objects 632, and therefore acts differently when disentangling a plurality of stacked medicine pieces.

The state in which the combined middle suspended object 1622 is held by the support arm 1621 will be described below (see Figs. 1B, 2, and 3). Relatively large swing operation of the lower end portion of the combined middle suspended object 1622 is allowed to mitigate a shock. For rotation about a virtual vertical axis, or rotation about its own axis, the swing width is suppressed to be small by the slit 1624a discussed above.

As illustrated in Figs. 2A to 2E, the middle suspended object 1623 according to a typical example configuration includes a vertically long flat plate portion 1623e, an upper end portion 1623a slightly projecting laterally from the upper end of the vertically long flat plate portion 1623e, a projecting portion 1623b projecting in the same direction as the upper end portion 1623a from the middle portion of the vertically long flat plate portion 1623e, and a lower end portion 1623c projecting to some longer extent from the lower end of the vertically long flat plate portion 1623e. The vertically long flat plate portion 1623e, the upper end portion 1623a, and the projecting portion 1623b extend in the first virtual plane PS1. On the contrary, the second portion 1623c2 of the lower end portion 1623c projects in the same direction as the direction in which the upper end portion 1623a and the projecting portion 1623b project, but is not located in the first virtual plane PS1 and is located in the second virtual plane PS2 which intersects the first virtual plane PS1. In this example, an obliquely crossing abutment surface 1623d of the lower end portion 1623c and the first virtual plane PS1 form an angle of about 30° therebetween.

The combined middle suspended object 1622 in Figs. 3A to 3E is obtained by stacking two middle suspended objects 1623 in Fig. 2. The combined middle suspended object 1622 is suspended over the annular upper end surface 23 of the outer rotary body 1020 with the upper end portion being attached to the support arm 1621 and with most portions of the two middle suspended objects 1623 being closely stacked on each other. One of the lower end portions 1623c of the two middle suspended objects 1623 includes the obliquely crossing abutment surface 1623d against which medicine pieces 5 abut.

### [Relationship of Interlocked Operation between Dimension Measurement Mechanism and Lateral Width Regulation Mechanism]

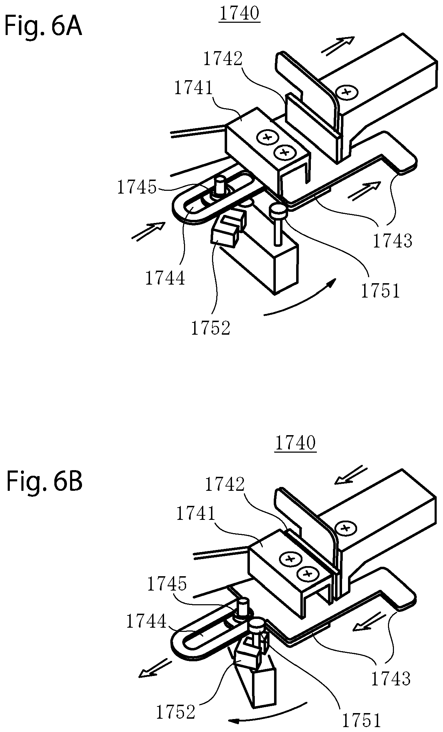

Fig. 5 illustrates the relationship between a dimension measurement mechanism 1740 and the lateral width regulation mechanism 1700. Figs. 6A and 6B are each a perspective view illustrating the appearance of the dimension measurement mechanism 1740. Figs. 7A to 7D are each a plan view illustrating a movable clamping portion 1742 of the dimension measurement mechanism 1740 and a link mechanism 73 of the lateral width regulation mechanism 1700 operating in conjunction with each other. A control portion 1800 illustrated in Fig. 5 controls the dimension measurement mechanism 1740 and the lateral width regulation mechanism 1700, and is constituted using a microprocessor etc. The control portion 1800 has a microprocessor as its core, and internally includes a height regulation portion 1801 configured to output a control instruction to the tablet height regulation mechanism 1600, a dimension measurement-lateral width regulation portion 1802 configured to output a control instruction to the dimension measurement mechanism 1740 and the lateral width regulation mechanism 1700, a rotational drive portion 1803 configured to output a control instruction to rotational drive motors 54a and 54b, a fallen medicine detecting portion 1804 configured to process a signal from the fallen medicine detecting means 56 and output a control signal, and first data storage means DM1 for storing first data and second data storage means MD2 for storing second data, which will discussed later.

In Fig. 5, the basic structural portion of the lateral width regulation mechanism 1700 (see the dotted portion) is obtained by improving the lateral width regulation mechanism 700 discussed earlier (see Fig. 13) which includes the link mechanism 73, the first regulation member 710, the second regulation member 720, and a bias spring known in the art. The lateral width regulation mechanism 1700 regulates the lateral width of the medicine transfer path on the annular upper end surface 23 of the outer rotary body 1020 and regulates the final height of medicine pieces, and is adapted to operate in conjunction with the dimension measurement mechanism 1740, which is now electrically driven, to be able to electrically variably adjust the lateral width of the medicine transfer path.

As illustrated in Figs. 6A and 6B, the dimension measurement mechanism 1740 includes: a fixed clamping portion 1741 (stationary portion); a movable clamping portion 1742 (movable portion) capable of advancing and retracting with respect to the fixed clamping portion 1741; a placement portion 1743 (transmission portion) and a long hole portion 1744 (guide portion) movable together with the movable clamping portion 1742; a fixed insertion portion 1745 (guide portion) inserted into a long hole of the long hole portion 1744 to regulate the moving direction of the placement portion 1743 and hence the moving direction of the movable clamping portion 1742; and a spring (not illustrated) configured to bias the placement portion 1743 in the direction of causing the movable clamping portion 1742 to abut against the fixed clamping portion 1741. In the free state, the fixed clamping portion 1741 and the movable clamping portion 1742 abut against each other (see Fig. 6B). When the movable clamping portion 1742 or the placement portion 1743 is pushed in a direction against the spring bias, the movable clamping portion 1742 is moved away from the fixed clamping portion 1741 (see Fig. 6A).

A motor 1750 (regulation drive member, dimension measurement drive member) configured to receive a control instruction from a dimension measurement-lateral width regulation portion 1802 of the control portion 1800 is disposed below the dimension measurement mechanism 1740 (see Fig. 5), as with the rotational drive motors 54a and 54b illustrated in Fig. 12. A swing member 1751 (transmission portion) (see Fig. 6A) operable to be swung according to rotational operation of the motor 1750 and an origin detection member 1752 (see Fig. 6B) configured to detect the swing member 1751 located at the origin are also disposed. When the motor 1750 is caused to operate in a predetermined direction, the gap between the two clamping portions 1741 and 1742 is widened. When the motor 1750 is rotated in reverse or in the free state, the gap between the two clamping portions 1741 and 1742 is narrowed to cause the two clamping portions 1741 and 1742 to abut against each other. This state is detected by the origin detection member 1752, which sends a detection signal to the control portion 1800.

Such a dimension measurement mechanism 1740 functions as a dimension measurement mechanism to clamp and measure the dimension of a medicine piece, and also functions as a mechanism to increase and decrease the amount of regulation on the lateral width of the medicine transfer path, under control by the dimension measurement-lateral width regulation portion 1802 of the control portion 1800 which is composed of a microprocessor etc., for example. The function as the dimension measurement mechanism (see Fig. 7) is implemented by moving the movable clamping portion 1742 with a fingertip etc. to move the fixed clamping portion 1741 and the movable clamping portion 1742 away from each other, placing a medicine piece 5a between the fixed clamping portion 1741 and the movable clamping portion 1742, and freeing the movable clamping portion 1742, thereby allowing the distance between the two members 1741 and 1742 to coincide with the dimension of the medicine piece 5a, and then measuring the distance as the dimension of the tablet (Figs. 7A and 7B).

While the distance may be measured by providing an electronic measuring instrument etc., the distance is inexpensively measured by the rotational drive portion 1803 of the control portion 1800 acquiring rotational phase data on the motor 1750 and converting such data into a distance in the present embodiment. For example, desired data can be obtained by actuating the motor 1750 with a drive force that is weaker than the spring bias force to cause the swing member 1751 to lightly abut against the placement portion 1743, and acquiring rotational phase data on the motor 1750 when the swing member 1751 is stopped by the abutment. In addition, measured values such as the lateral width (see Fig. 7A) of the medicine piece 5a, the thickness (see Fig. 7B) of the medicine piece 5a, and the length (not illustrated) of the medicine piece 5a are acquired by the control portion 1800, and held by the first data storage means DM1 as first data.

In this manner, the control portion 1800 can acquire dimension data on the medicine piece 5a placed on the dimension measurement mechanism 1740 by controlling the motor 1750. When the medicine piece 5a is removed from the dimension measurement mechanism 1740 after the dimension measurement (see Fig. 7C), the movable clamping portion 1742 is moved to the fixed clamping portion 1741 by the spring bias. By actuating the motor 1750 with a drive force that is stronger than the spring bias force according to a control instruction from the control portion 1800, the movable clamping portion 1742 can be moved to a position corresponding to the lateral width of the medicine piece 5a or other positions (see Fig. 7D), even in the absence of the medicine piece 5a.

A support portion for the movable clamping portion 1742 or the rear end portion of the placement portion 1743 is coupled to the front end portion of the link mechanism 73 to form a pair. Therefore, when the movable clamping portion 1742 is moved by control by the control portion 1800 and drive by the motor 1750, the link mechanism 73 is advanced and retracted in the longitudinal direction in conjunction with such movement. Consequently, the lateral width of the medicine transfer path which is regulated by the first and second regulation members 710 and 720, that is, the lateral width allowed for medicine pieces placed on the annular upper end surface 23 of the outer rotary body 1020 to be moved, is increased and reduced from the outer peripheral side as discussed earlier. The amount of regulation on the lateral width of medicine pieces on the annular upper end surface 23 can be increased and decreased under control by the control portion 1800, even in the absence of the medicine piece 5a on the dimension measurement mechanism 1740. Therefore, the medicine piece 5a placed on the dimension measurement mechanism 1740 in order to determine the regulation on the lateral width of medicine pieces on the annular upper end surface 23 by the first and second regulation members 710 and 720 can be immediately dispensed without being wasted.

The dimension measurement-lateral width regulation portion 1802 (see Fig. 5) of the control portion 1800 (control portion of medicine feeder) outputs a control instruction for setting the width (lateral width of medicine transfer path) of the medicine transfer path on the annular upper end surface 23 of the outer rotary body 1020 to an appropriate width such as a lateral width that coincides with the lateral width of medicine pieces, an approximate width calculated by multiplying the lateral width of medicine pieces by a predetermined coefficient, or a lateral width that is slightly less than such a width by actuating the motor 1750 of the dimension measurement mechanism 1740 and hence the first and second regulation members 710 and 720 of the lateral width regulation mechanism 1700 using the value (medicine dimension data) of the lateral width of medicine pieces measured by the dimension measurement mechanism 1740. This is one of initial setting processes performed before discharge of medicine pieces is started. Rotational speed control, detection of discharge of medicine pieces, etc. discussed earlier are also performed during operation to discharge medicine pieces.

In addition, the control portion 1800 is configured to detect the next congestion of medicine pieces and loosen the width of the medicine transfer path (increase the lateral width of the medicine transfer path), as necessary, also during operation to discharge medicine pieces, although such procedures are not complicated and thus are omitted from a flowchart. Congestion of medicine pieces is detected based on a detection signal from the fallen medicine detecting means 56. It is determined that "congestion of medicine pieces has not occurred" when the time interval of detection timing for medicine pieces having fallen from the fall-discharge port 14 does not reach a predetermined congestion-time interval. Then, it is determined that "congestion of medicine pieces has occurred" when the time interval reaches the congestion-time interval. Here, the congestion-time interval is a setting value of the time interval determined in advance by adding an increase amount for erroneous determination prevention to a normal-time interval known through trial operation etc.

Increasing the lateral width of the medicine transfer path corresponds to loosening the regulation on the lateral width for medicine pieces on the medicine transfer path. Specifically, the width of the medicine transfer path is increased by decreasing the distance by which the distal ends of lower portions 711 and 721 (see Fig. 13) of the first and second regulation members 710 and 720 project from the outer peripheral side toward the inner peripheral side to the greatest degree immediately above the annular upper end surface 23 of the outer rotary body 1020. In the medicine feeder 1100 (see Fig. 5), most operation is performed in the absence of the medicine piece 5a on the dimension measurement mechanism 1740 (see Figs. 7C and 7D) . When the control portion 1800 actuates the motor 1750, the movable clamping portion 1742 of the lateral width regulation mechanism 1700 is advanced and retracted, and the link mechanism 73 and further the first and second regulation members 710 and 720 are accordingly moved. The motor 1750 for dimension measurement is also used to regulate the lateral width by using such operation.

Further, the dimension measurement-lateral width regulation portion 1802 of the control portion 1800 can increase the lateral width stepwise, rather than monotonously. The number of steps, the amount of increase for each step, the time for each step, etc. can be specified by initial settings of parameters, setting changes, etc. For example, a case where the number of steps is set to 4, the amount of increase for each step is uniformly set to 5%, and the time for each step is uniformly set to 3 seconds is assumed. When the occurrence of congestion of medicine pieces is detected, the control portion 1800 first increases the lateral width of the medicine transfer path at the relevant location to 105% of the initial width by loosening the width regulation by 5% after the lapse of 3 seconds . The width regulation is further loosened by 5% after the lapse of additional 3 seconds to increase the lateral width of the medicine transfer path at the relevant location to 110% of the initial width.

After that, the width regulation is further loosened by 5% after the lapse of additional 3 seconds to increase the lateral width of the medicine transfer path at the relevant location to 115% of the initial width, and the width regulation is further loosened by 5% after the lapse of additional 3 seconds to increase the lateral width of the medicine transfer path at the relevant location to 1200 of the initial width. In order to avoid an undesirable state in which medicine pieces are arranged side by side, there may be a constraint that the lateral width of the medicine transfer path must be less than 1.5 times the lateral width of medicine pieces, even when the regulation is loosened to the greatest degree . When the fallen medicine detecting means 56 detects discharge of medicine pieces from the fall-discharge port 14 sometime in the course of loosening the regulation on the lateral width as illustrated in Fig. 12, the fallen medicine detecting portion 1804 of the control portion 1800 returns the first and second regulation members 710 and 720 to the original setting positions and ends the loosening of the regulation on the lateral width. When the time further elapses and the process times out, the fallen medicine detecting portion 1804 determines that the medicine feeder 1100 has been emptied.

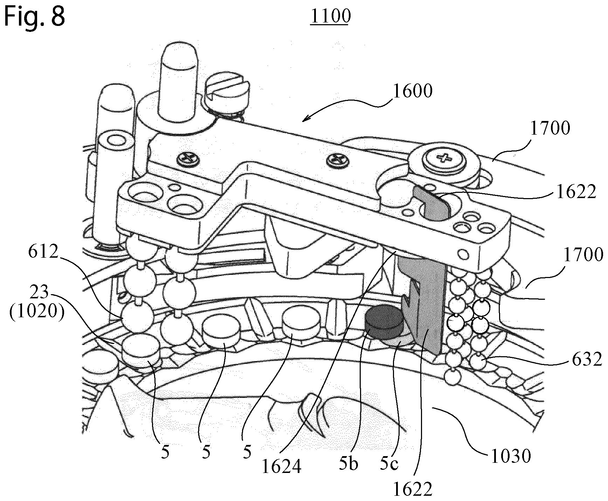

The use and operation of the medicine feeder 1100 will be described with reference to the drawings . Fig. 8 is a perspective view illustrating the appearance of the medicine feeder 1100 in the operating state, in particular the tablet height regulation mechanism 1600 in the operating state, including the tablet height regulation mechanism 1600 classifying medicine pieces 5b and 5c that have come to the combined middle suspended object 1622, among the medicine pieces 5. Fig. 9 illustrates the combined middle suspended object 1622 classifying the medicine pieces 5b and 5c in the swung state, in which Fig. 9A is a plan view and Fig. 9B is a front view. Before using the medicine feeder 1100 for medicine dispensation, it is necessary to cause the control portion 1800 to hold at least the lateral width and the thickness among shape data on medicine pieces. Dimension measurement and data setting are performed, although such processes may be omitted if data have been input in advance. Dimension measurement may be performed by a different device, and data on the dimension values may be manually input to the medicine feeder 1100. When the medicine feeder 1100 is caused to operate in a lateral width measurement mode after the medicine piece 5a is set in a lateral posture on the dimension measurement mechanism 1740 (see Fig. 7A), however, dimension measurement and data setting for the lateral width of the medicine piece 5a are automatically performed (first data storage means DM1 in Fig. 5). When the medicine feeder 1100 is caused to operate in a thickness measurement mode after the medicine piece 5a is set in a vertical posture on the dimension measurement mechanism 1740 (see Fig. 7B), dimension measurement and data setting for the thickness of the medicine piece are automatically performed (see Fig. 5) . After that, the medicine piece 5a is taken out of the dimension measurement mechanism 1740 and put into rotary containers. The medicine pieces thus become ready for dispensation (see Fig. 7C).

Next, for the tablet height regulation mechanism 1600, when the medicine feeder 1100 is caused to operate in a preparation mode, the height regulation portion 1801 of the control portion 1800 causes the motor 1630 to operate based on thickness data on the medicine piece, and accordingly the tablet height regulation mechanism 1600 elevates and lowers the elevating mechanism 1631 to adapt the height of the front suspended objects 612, the combined middle suspended object 1622, and the rear suspended objects 632 to the thickness of the medicine piece 5a. For the lateral width regulation mechanism 1700, the height regulation portion 1801 of the control portion 1800 causes the motor 1750 to operate based on lateral width data on the medicine piece 5a, and accordingly the lateral width regulation mechanism 1700 operates to swing the first and second regulation members 710 and 720 (Fig. 13) to thereby adapt the lateral width at the relevant locations of the medicine transfer path on the annular upper end surface 23 of the outer rotary body 1020 to the lateral width of the medicine piece.

Next, a necessary number or a greater number of medicine pieces 5 are put into the medicine feeder 1100, specifically medicine pieces 5 are put onto the inner inclined rotary body 1030 surrounded by the outer rotary body 1020, and the medicine feeder 1100 is caused to operate in a medicine dispensing mode. The medicine pieces 5 are lifted by the inner inclined rotary body 1030 one after another to be placed onto the annular upper end surface 23 (medicine transfer path) of the outer rotary body 1020 (see Fig. 8), and moved to pass under the front suspended objects 612, the middle suspended objects 1623, and the rear suspended objects 632 in this order along with rotation of the outer rotary body 1020.

At that time, medicine pieces 5 placed on the annular upper end surface 23 alone without being stacked on each other hardly interfere with the front and rear suspended objects 612 and 632, or are only slightly moved on the annular upper end surface 23 even if the medicine pieces 5 interfere with the suspended objects 612 and 632, and the medicine pieces 5 pass under the middle suspended objects 1623 between the suspended objects 612 and 632 without interfering with the middle suspended object 1623 at all. For the medicine pieces 5b and 5c vertically stacked on each other (see Fig. 8), on the contrary, the upper medicine piece 5b may abut against the front suspended objects 612, and the stacked state of the medicine pieces may be broken by the repulsion force. The stacked state of the medicine pieces may not be broken but remain, since the front suspended objects 612 which are deformable when the lower end portions of the front suspended objects 612 are pushed sideways weakly abut not to damage the medicine pieces 5.

When the medicine pieces 5b and 5c having passed through the front suspended objects 612 while remaining in the state of being vertically stacked on each other are carried to the combined middle suspended object 1622 (see Fig. 9), the upper medicine piece 5b abuts against the obliquely crossing abutment surface 1623d of the lower end portion 1623c of the combined middle suspended object 1622, and is not allowed to move straight forward by the repulsion force. Thus, the upper medicine piece 5b is moved along the obliquely crossing abutment surface 1623d of the second portion 1623c2 of the lower end portion 1623c or the surface of the second portion 1623c1. Since the moving direction of the upper medicine piece 5b is directed toward the inner peripheral side of the outer rotary body 1020 in this manner, the upper medicine piece 5b travels inward from the annular upper end surface 23 of the outer rotary body 1020, and often falls onto the inner inclined rotary body 1030 (see the single-dot and dash line in Fig. 9B).

The lower medicine piece 5c often passes under the combined middle suspended object 1622, but occasionally falls from the annular upper end surface 23 of the outer rotary body 1020 together with the medicine piece 5b. The combined middle suspended object 1622 is not deformed when the lower end portion of the combined middle suspended object 1622 is pushed sideways, and thus is better able to disentangle stacking of the medicine pieces 5b and 5c than the other suspended objects 612 and 632. Since the combined middle suspended object 1622 is configured to swing to mitigate a shock caused at the time of abutment with the medicine pieces or a pressure generated thereafter in order not to damage the medicine pieces, the medicine pieces 5b and 5c may push away and pass through the combined middle suspended object 1622 while remaining in the stacked state though on rare occasions. In that case, the rear suspended objects 632 exert the function of gently disentangling stacked medicine pieces, and only when the stacked state is not broken, the lateral width regulation mechanism 1700 on the rear side discussed earlier exerts the function of regulating the lateral width of the stacked medicine pieces 5b and 5c by reducing the width of the medicine transfer path on the annular upper end surface 23 of the outer rotary body 1020 from the outer peripheral side, in addition to gently regulating the height of the stacked medicine pieces 5b and 5c. Therefore, stacking of the medicine pieces 5b and 5c is resolved, or the medicine pieces 5b and 5c are forcibly moved onto the inner inclined rotary body 1030 from the top of the annular upper end surface 23 of the outer rotary body 1020 while remaining in the stacked state. Thus, the stacked medicine pieces 5b and 5c are not fed from the medicine transfer path into the fall-discharge port 14.

Operation of the combined middle suspended object 1622 in which the two middle suspended objects 1623 are disposed in close contact with each other will be described with reference to Figs . 9A to 9C. The abutment-interference portion of the combined middle suspended object 1622 is inclined with respect to the travel direction (see the double-dot and dash line) of the stacked medicine pieces 5b and 5c due to the outer rotary body 1020. Specifically, the obliquely crossing abutment surface 1623d (abutment surface and guide surface in the front half) of the combined middle suspended object 1622 is tilted by an angle of θ1, while the vertically long flat plate portion 1623e (guide surface in the rear half) of the combined middle suspended object 1622 is tilted by an angle of only θ2, the angle θ1 being larger than the angle θ2 (θ1 > θ2). Thus, a stronger breaking force acts in the front half on the stacked medicine pieces 5b and 5c which abut against the combined middle suspended object 1622, while an enhanced force acts in the rear half to move the medicine piece 5b out onto the inner inclined rotary body 1030 from the top of the outer rotary body 1020, which increases the possibility that the stacked medicine pieces do not remain on the medicine transfer path.

Moreover, while the two middle suspended objects 1623 and 1623 which compose the combined middle suspended object 1622 are in close contact with each other in the free state (see Fig. 3B), the two middle suspended objects 1623 and 1623 are separable. Thus, when the medicine piece 5b abuts against the obliquely crossing abutment surface 1623d (see Fig. 9), the upper end portions 1623a are hardly moved, and the lower end portions 1623c (1623d) are swung, and slightly slid with respect to each other and repeatedly separated from and brought into contact with each other at the same time, thereby reducing and mitigating shock caused at the time of abutment or a friction force generated thereafter. Consequently, stacking of the medicine pieces 5b and 5c is readily resolved at a high probability, and the medicine pieces 5 are caused to fall to be discharged from the fall-discharge port 14 one by one.

When congestion of medicine pieces occurs at the first regulation member 710 or the second regulation member 720 during consecutive discharge of medicine pieces, even if on rare occasions, detection of fallen medicine pieces by the fallen medicine detecting means 56 is interrupted, and the fallen medicine detecting portion 1804 of the control portion 1800 detects a time-out due to such interruption. The operation to mitigate the lateral width of the medicine transfer path discussed above is performed according to control by the fallen medicine detecting portion 1804 of the control portion 1800 based on such detection. As a result, congestion of medicine pieces which previously could not be immediately resolved can often be resolved before a time-out, which allows automatically discharging medicine pieces at a high possibility with no medicine pieces being left.

### [Medicine Dispensing System]

Fig. 10 illustrates the specific configuration of a medicine dispensing system in which the medicine feeder 1100 according to the present invention is used. Fig. 10A is a schematic view illustrating the configuration of the medicine dispensing system, also illustrating upper lids of a swing open-close type for the medicine feeders 1100. Fig. 10B is a front view of a medicine feeder 2110 of a cassette mount-unmount type with a cassette being removed. Fig. 5 includes a perspective view illustrating the appearance of mechanical portions of the medicine feeder 1100 and a functional block diagram of the control portion 1800 (control portion of medicine feeder) as discussed above. The matter about the medicine feeder 1100 that was not mentioned in the description of the components in Fig. 5 will be described. The control portion 1800 of the medicine feeder 1100 (control portion of medicine feeder) is communicably connected to a controller 2300 of a medicine dispensing apparatus 2000 (control portion of medicine dispensing apparatus), in which the medicine feeder 1100 is incorporated, through a LAN cable etc. (see the single-dot and dash line with arrows in Fig. 10A) to be able to transmit and receive data to and from the controller 2300. When medicine dimension data including default values of the lateral width and the thickness of medicine pieces are received from the controller 2300 as illustrated in Fig. 5, the medicine dimension data, in particular the values of the lateral width and the thickness of the medicine pieces among such data, are held in the second data storage means DM2 as second data.

As illustrated in Fig. 5, when an instruction to execute dimension measurement is received from the controller 2300 of the medicine dispensing apparatus 2000 illustrated in Fig. 10A, the control portion 1800 of the medicine feeder 1100 causes the dimension measurement mechanism 1740 which also serves as a dimension measurement mechanism to perform dimension measurement operation. The control portion 1800 holds first data (medicine dimension data; measured values of the lateral width and the thickness of medicine pieces) acquired through the medicine dimension measurement in the first data storage means DM1 as discussed above. In addition, the control portion 1800 transmits the first data to the relevant controller 2300 which executes data transmission of the second data discussed above.

Further, after the first data are acquired and held, the medicine feeder 1100 controls the motors 1630 and 1750 based on the medicine dimension data, in order to cause the lateral width regulation mechanism 1700 to regulate the lateral width of the medicine transfer path on the annular upper end surface 23 of the outer rotary body 1020, and cause the tablet height regulation mechanism 1600 to regulate the passable height of medicine pieces on the annular upper end surface 23 of the outer rotary body 1020. Additionally, also after the second data are acquired and held, the medicine feeder 1100 controls the motors in the same manner based on the medicine dimension data, in order to cause the lateral width regulation mechanism 1700 to regulate the lateral width of the medicine transfer path, and cause the tablet height regulation mechanism 1600 to regulate the height of medicine pieces on the medicine transfer path that can pass.

A medicine dispensing server 3000 in Fig. 10 can transmit and receive data to and from a prescription ordering system OS on the upper level and a medicine dispensing apparatus 2000 on the lower level via a LAN etc. By executing a program, the medicine dispensing server 3000 receives prescription data from the prescription ordering system OS and holds such data, and issues a medicine dispensation instruction to a relevant one of the medicine dispensing apparatus 2000 and other medicine dispensing apparatuses (not illustrated), if any, based on the prescription data. When the medicine dispensing server 3000 obtains data indicating the operating state of the medicine dispensing apparatus 2000 from the medicine dispensing apparatus 2000 as an upper-level device for the medicine dispensing apparatus 2000, the medicine dispensing server 3000 holds such data as the dispensing apparatus status. The medicine dispensing server 3000 also holds a medicine master composed of an appropriate database that is easy to search and update. Known medicine information on various types of medicine has been registered in advance in the medicine master.

Further, the medicine master in the medicine dispensing server 3000 has been enhanced to be able to hold data such as the lateral width and the height of each medicine piece as a part of the medicine information. A data holding region for lateral width values and height values is secured in the medicine master for each piece of the medicine information. When a lateral width value and a height value are already found and registered, data on such values are held. In the medicine master, when a lateral width value or a height value of a medicine piece is not found yet, a data holding region for holding data on such values is secured, or tentative values indicating an undetermined state are set.

When a medicine dispensation instruction is issued to the medicine dispensing apparatus 2000, the medicine dispensing server 3000 issues a medicine dispensation instruction including data on found values (medicine dimension data; default values of the lateral width and the thickness of medicine pieces) of the lateral width and the height of medicine pieces, if data on such values are held, when acquiring information on medicine pieces to be dispensed from the medicine master. When such data are not held in the medicine master, however, a medicine dispensation instruction not including the data is transmitted from the medicine dispensing apparatus 2000 to the controller 2300. When the medicine dispensing server 3000 receives the first data (medicine dimension data; measured values of the lateral width and the thickness of medicine pieces) transmitted from the controller 2300 of the medicine dispensing apparatus 2000, conversely, the medicine dispensing server 3000 registers such data in the medicine master as found values for the relevant medicine pieces.

The medicine dispensing apparatus 2000 includes the controller 2300 (control portion) stored in an electrical equipment space in the housing, a fallen medicine collection mechanism, a packing machine, etc. stored in the lower part of the housing, and a plurality of medicine feeder storages 2100 (of a type exclusively for specific tablets) mounted in the upper part of the housing to be able to be individually drawn out. The medicine feeder storage 2100 includes a large number of medicine feeders 2110 (of a type exclusively for specific tablets) of a cassette mount-unmount type, and a fall guide mechanism configured to guide medicine pieces discharged from the medicine feeders 2110 to the fallen medicine collection mechanism. As illustrated in Fig. 10B, the medicine feeder 2110 is composed of a set of a fixed base 2112 and a removable cassette 2111. When a motor in the base 2112 is actuated under control by the controller 2300 with the cassette being mounted, the medicine feeder 2110 drops and discharges medicine pieces one by one when the cassette 2111 is driven.

The medicine dispensing apparatus 2000 also includes a plurality of medicine feeders 1100 of a type securely installed as a whole with a container portion and a base being integrated with each other, unlike the medicine dispensing apparatus discussed earlier. While it is not essential that a plurality of medicine feeders 1100 should be mounted, it is more convenient when a plurality of medicine feeders 1100 are provided to allow selective use than when a single medicine feeder 1100 is provided. In the illustrated example, a plurality of (in the drawing, vertically arranged three rows of) medicine feeder shelves 2200 are incorporated in a part (right end portion in the drawing) of the upper portion of the housing to substitute a part of the medicine feeder storages 2100. Further, a plurality of (four in the drawing) medicine feeders 1100 (of a type that adapts to many types of tablets) are mounted in each of the medicine feeder shelves 2200 to be arranged in line in the front-rear direction.

The medicine dispensing apparatus 2000 also includes a touch panel 2400 mounted to exert the function of allowing operation input and screen display under control by the controller 2300. When a medicine dispensation instruction is received from the medicine dispensing server 3000, the controller 2300 of the medicine dispensing apparatus 2000 performs automatic processing in a possible range, and displays a screen for guidance etc. for a medicine dispensing person, as necessary, and receives an instruction from the medicine dispensing person. For example, when a medicine feeder 1100 of a type that adapts to many types of tablets is selected for execution of medicine dispensation, the medicine dispensing apparatus 2000 displays an instruction to put medicine pieces into the relevant medicine feeder 1100 on the screen, or displays procedures for assistive work for the relevant medicine feeder 1100 to measure the lateral width and the thickness of medicine pieces, if help from the medicine dispensing person is necessary.

The use and operation of the medicine dispensing system will be described. A medicine dispensation instruction is transmitted from the medicine dispensing server 3000 to the medicine dispensing apparatus 2000 by operating the touch panel 2400 etc. When the medicine dispensation instruction is received by the controller 2300 of the medicine dispensing apparatus 2000, the content of the medicine dispensation instruction is displayed on the touch panel 2400 for confirmation. When medicine pieces indicated in the medicine dispensation instruction to be automatically dispensed are stored in the medicine feeder 2110 of the medicine feeder storage 2100, the medicine pieces are automatically allocated appropriately by the controller 2300 to complete preparation. When the medicine dispensing person makes a confirmation by operating the touch panel 2400, the medicine pieces to be dispensed are automatically discharged from the medicine feeder 2110, collected while falling to reach a packing device, and separately stored in dispensing paper.

When medicine pieces to be automatically dispensed can be handled by some of the medicine feeders 2110 but are not stored in any of the medicine feeders 2110. A prompt is displayed on the display panel 2400 to urge the medicine dispensing person to store the target medicine pieces in the relevant medicine feeder 2110 or select some of the medicine feeders 1100 and store the target medicine pieces in the selected medicine feeder 1100. When the medicine dispensing person selects the medicine feeder 2110, automatic medicine dispensation is performed as discussed above when a panel operation for confirmation is performed after the target medicine pieces are put into the medicine feeder 2110.

When the medicine dispensing person selects the medicine feeder 1100, on the contrary, a confirmation operation is performed after the target medicine pieces are put into the medicine feeder 1100 as discussed later. When the medicine pieces to be automatically dispensed cannot be handled by any of the medicine feeders 2110 or when the medicine feeders 2110 that can handle the medicine pieces to be automatically dispensed are already occupied by other medicine pieces that cannot be mixed because of differences in the effect etc., medicine feeders 1100 that can be allocated at that time are displayed on the screen of the touch panel 2400 in a list or map format. The medicine dispensing person first selects an appropriate medicine feeder 1100 through a screen operation. The controller 2300 of the medicine dispensing apparatus 2000 performs a setup process for the medicine feeder 1100 according to the selection. Since the content of the process is different according to whether or not dimension data on the medicine pieces to be automatically dispensed are held in the medicine master of the medicine dispensing server 3000, two cases will be separately described here. When dimension data on the medicine pieces to be automatically dispensed are already held in the medicine dispensing server 3000, the relevant medicine dimension data are included in a medicine dispensation instruction to be transmitted to the controller 2300 of the medicine dispensing apparatus 2000, and further transferred to the relevant medicine feeder 1100. The tablet height regulation mechanism 1600 and the lateral width regulation mechanism 1700 of the medicine feeder 1100 automatically regulates the height and the lateral width of medicine pieces so as to be passable on the medicine transfer path based on the transferred medicine dimension data.

Concurrently with the setup process, the medicine dispensing apparatus 2000 displays not only the name and the number of medicine pieces to be prescribed, but also the medicine dimension data, on the touch panel 2400. In addition, operation to unlock a lock mechanism (not illustrated) is also performed, which enables the relevant medicine feeder shelf 2200 to be drawn out. When this is indicated to the medicine dispensing person by flashing on and off an LED (not illustrated) etc., the medicine dispensing person draws out the relevant medicine feeder shelf 2200, and opens the upper lid of the relevant medicine feeder 1100 [the second medicine feeder 1100 from the front side of the lowermost medicine feeder shelf 2200 in Fig. 10B] clearly indicated by turning on an individual LED (not illustrated) etc.

The medicine dispensing person can visually, although roughly, confirm the completion of automatic setup performed using the medicine dimension data by seeing the position indicated on the scale of the dimension measurement mechanism 1740 on the front surface of the medicine feeder 1100 or seeing the positions of the regulation members 710 and 720 and the suspended objects 612, 1623, and 632. After that, the medicine dispensing person puts the medicine pieces to be automatically dispensed into a space above the inner inclined rotary body 1030 of the relevant medicine feeder 1100, and closes the upper lid when the input of a necessary amount of medicine pieces is finished. Further, when the medicine dispensing person pushes the medicine feeder shelf 2200 which has been drawn out back into the housing of the medicine dispensing apparatus 2000, it is automatically indicated on the screen of the touch panel 2400 that preparation for automatic medicine dispensation has been completed. After confirming the indication of the completion of preparation, the medicine dispensing person causes the medicine dispensing apparatus 2000 to start automatic medicine dispensation by performing a screen operation etc. on the touch panel 2400.

When dimension data on the medicine pieces to be automatically dispensed are not held in the medicine dispensing server 3000 yet, on the other hand, the process is performed as follows. First, work procedures for measuring the lateral width and the thickness of medicine pieces are displayed on the touch panel 2400 under control by the controller 2300 of the medicine dispensing apparatus 2000 which has confirmed that medicine dimension data are not included in the medicine dispensation instruction from the medicine dispensing server 3000 to the medicine dispensing apparatus 2000. When the medicine dispensing person performs a screen operation on the touch panel 2400 after placing a medicine piece on the dimension measurement mechanism 1740 of the medicine feeder 1100 according to the display, one of the lateral width and the thickness of the medicine piece is measured (see Fig. 7A). Next, when the same operation is performed after changing the posture of the medicine piece (see Fig. 7B), the other of the lateral width and the thickness of the medicine piece is measured.

When dimension measurement with human intervention is finished, medicine dimension data that are necessary to set up the medicine feeder 1100 are held in the first data storage means DM1 of the control portion 1800 of the medicine feeder 1100. Moreover, the medicine dimension data are transmitted from the control portion 1800 of the medicine feeder 1100 to the controller 2300 of the medicine dispensing apparatus 2000, and further transferred to the medicine dispensing server 3000 to be held as data in the medicine master in association with the relevant medicine pieces. Therefore, automatic setup can thereafter be performed using the medicine dimension data for the same medicine pieces by not only the medicine feeder 1100 which has performed dimension measurement, but also any of the large number of medicine feeders 1100 mounted on any of the medicine dispensing apparatuses that transmit and receive data to and from the medicine dispensing server 3000 which holds the data.

When dimension measurement with human intervention is finished, the same state is established as the state discussed above in which automatic setup has been completed for a case where medicine dimension data are held by the medicine dispensing server 3000. Thus, the medicine dispensing person thereafter puts medicine pieces to be automatically dispensed into the space above the inner inclined rotary body 1030, and closes the upper lid after input of a necessary amount of medicine pieces is finished, in the same manner as discussed above. Further, when the medicine dispensing person pushes the medicine feeder shelf 2200 which has been drawn out back into the housing of the medicine dispensing apparatus 2200, an indication of the completion of preparation for automatic medicine dispensation is automatically displayed on the screen of the touch panel 2400. After confirming the indication, the medicine dispensing person causes the medicine dispensing apparatus 2000 to start automatic medicine dispensation through a screen operation etc. After the start of automatic medicine dispensation, an appropriate amount of medicine pieces is discharged from appropriate medicine feeders 2110 and 1100 according to a prescription instruction in any case, and further the discharged medicine pieces are separately packed by the packing device.

### [Others]

In the above embodiment, when medicine dimension measurement is performed by the medicine feeder 1100, obtained medicine dimension data are transmitted to the medicine dispensing server 3000 by way of the medicine dispensing apparatus 2000 so that the medicine dimension data can be repeatedly used thereafter. However, it is not necessary that the medicine dimension data held by the medicine dispensing server 3000 should not be the same. For example, the medicine dimension data may be manually finely adjusted or reset through a terminal operation etc. of the medicine dispensing server 3000, or medicine dimension measurement may be performed a plural number of times successively or intermittently, where the number of times is specified by a parameter etc., to adopt an average value as the medicine dimension data.

In the above embodiment, examples of the items to be displayed on the touch panel 2400 include guidance for the medicine dispensing person, an instruction to put medicine pieces into the relevant medicine feeder 1100, and procedures for assistive work for measuring the lateral width and the thickness of medicine pieces in the relevant medicine feeder 1100 . Other examples to be displayed may include whether or not procedures for the medicine dispensing person related to the relevant medicine feeder 1100 are appropriate, the operation status related to recovery operation to discharge medicine pieces at a high speed performed with the relevant medicine feeder 1100 being drawn forward out of the housing of the medicine dispensing apparatus 2000, etc. The operation status related to test operation to discharge medicine pieces at a normal speed in the same draw-out state may also be displayed. In that case, the presence or absence of the necessity to finely adjust medicine dimension data and the degree of such necessity can be relatively easily grasped by monitoring or confirming display on the screen or the operation status of the actual device.

In the above embodiment, the medicine dispensing apparatus 2000 is not equipped with a manual medicine dispensing unit (see Japanese Unexamined Patent Application Publication No. 2007-297066, JPA 2007-297066, for example). However, a manual medicine dispensing unit may be provided around the lower end portion of the upper portion of the housing of the medicine dispensing apparatus 2000, as in many medicine dispensing apparatuses. In that case, when the controller 2300 selects a unit to discharge medicine pieces, the number of types of medicine pieces that can be easily handled by the medicine dispensing apparatus 2000 can be further increased by sequentially performing the processes of preferentially selecting the medicine feeder 2110 if possible, selecting the medicine feeder 1100 if the medicine feeder 2110 cannot be selected, and selecting the manual medicine dispensing unit if either medicine feeder cannot be selected.

In the above embodiment, one set of mechanical portions 1020 to 1752 and one control portion 1800 are incorporated in each medicine feeder 1100. When the control portion 1800 has a sufficiently high processing capability, a sufficiently large memory capacity, and a sufficiently large number of I/O ports, however, one or a small number of control portions 1800 may be configured to perform control etc. related to a greater number of mechanical portions 1020 to 1752 by mounting a multi-task control program on each control portion 1800 etc.

In the above embodiment, the amount of mitigation of the lateral width of the medicine transfer path is increased repeatedly four times by 5% each time at the time of medicine congestion. However, the number of repetitions is not limited to four, and may be more or less than that. The amount of adjustment per time is also not limited to 5%, and may be more or less than that. The manner of increase is also not limited to a monotonous increase, and the lateral width may be increased by 6% and then decreased by 3% repeatedly, for example, in order to enhance resolution of congestion in the row of medicine pieces while suppressing the total amount of mitigation.

In the above embodiment, a large number of medicine pieces 5 are randomly put into the medicine feeder 1100 at a timing later than both the height adjustment related to the tablet height regulation mechanism 1600 and the lateral width adjustment related to the lateral width regulation mechanism 1700. However, this order is not essential. The input of a large number of medicine pieces to the medicine feeder 1100, the height adjustment related to the tablet height regulation mechanism 1600, and the lateral width adjustment related to the lateral width regulation mechanism 1700 may be performed in any order before the medicine feeder 1100 is caused to operate in the medicine dispensing mode.

In the above embodiment, only the support arm 1621 of the second regulation member 1620 is a regulation member extending over the lateral width regulation mechanism 1700, and the support arm 1611 of the first regulation member 1610 is not such an extending regulation member. However, this is not essential, and the support arm 1611 of the first regulation member 1610 may also be a regulation member extending over the lateral width regulation mechanism 1700.

### INDUSTRIAL APPLICABILITY

The medicine feeder according to the present invention may be used to replace some or all of a large number of medicine feeders of a rotary alignment disk type mounted on a tablet dispensing apparatus, may be mounted on a tablet splitting device on which only one or a small number of medicine feeders are mounted, and further may be mounted on a tablet counter (medicine piece counter) etc. operable to count up the number of medicine pieces consecutively fed in a device operable to charge medicine pieces such as tablets into a medicine bottle.

## Claims

1. A medicine feeder comprising:
an outer rotary body including an internal space having an opening portion opening upward and an annular upper end surface surrounding the opening portion, the outer rotary body being rotatable about a virtual vertical line extending in a vertical direction in the internal space;
an inner inclined rotary body disposed in the internal space of the outer rotary body, the inner inclined rotary body being rotatable about a virtual inclined line tilted with respect to the vertical line, with a plurality of solid medicine pieces being placed on an upper surface portion of the inner inclined rotary body, to move the plurality of medicine pieces onto the annular upper end surface of the outer rotary body while rotating; and
an alignment regulation mechanism configured to align the plurality of medicine pieces, which have been moved onto the annular upper end surface of the outer rotary body, in a rotational direction of the annular upper end surface when the outer rotary body is rotating, wherein:
the alignment regulation mechanism includes a tablet height regulation mechanism including a plurality of suspended objects suspended over the annular upper end surface, the tablet height regulation mechanism being configured to regulate heights of the medicine pieces using the plurality of suspended objects;
the plurality of suspended objects include at least one deformable suspended object that is deformed when a lower end portion of the deformable suspended object is pushed sideways and at least one non-deformable suspended object that is not deformed when a lower end portion of the non-deformable suspended object is pushed sideways with an upper end portion of the non-deformable suspended object being swingably supported;
the medicine feeder further includes a control portion configured to control rotation of the outer rotary body and fallen medicine detecting means for detecting a fallen medicine piece that has been carried to a fall-discharge port by the outer rotary body;
the alignment regulation mechanism includes a lateral width regulation mechanism configured to regulate a lateral width of a medicine transfer path on the annular upper end surface of the outer rotary body;
the lateral width regulation mechanism is configured to adjust a regulation amount of the lateral width according to a control instruction from the control portion;
the lateral width regulation mechanism increases the lateral width according to the control instruction from the control portion when an interval of detection for fallen medicine pieces by the fallen medicine detecting means reaches a congestion-time interval which is longer than a normal-time interval;
the medicine feeder further includes a dimension measurement mechanism operable to clamp a medicine piece and measure a dimension of the medicine piece and a dimension measurement drive mechanism configured to move a movable portion of the dimension measurement mechanism;
the lateral width regulation mechanism is configured to set an initial value of the lateral width in conjunction with the movable portion of the dimension measurement mechanism;
when the control portion acquires medicine dimension data on the medicine piece placed on a dimension measurement portion of the dimension measurement mechanism by driving the dimension measurement drive mechanism, the control portion outputs the control instruction to cause the lateral width regulation mechanism to regulate the lateral width based on the acquired medicine dimension data;
the tablet height regulation mechanism includes an elevating mechanism operable to elevate and lower the plurality of suspended objects;
the medicine feeder further includes first data storage means for holding the medicine dimension data acquired by the dimension measurement mechanism, and second data storage means for acquiring and holding medicine dimension data transmitted from an upper-level device; and
the lateral width regulation mechanism is caused to regulate the lateral width and the elevating mechanism of the tablet height regulation mechanism is caused to regulate the height based on the medicine dimension data held by one of the first data storage means and the second data storage means.

2. A medicine feeder comprising:
an outer rotary body including an internal space having an opening portion opening upward and an annular upper end surface surrounding the opening portion, the outer rotary body being rotatable about a virtual vertical line extending in a vertical direction in the internal space;
an inner inclined rotary body disposed in the internal space of the outer rotary body, the inner inclined rotary body being rotatable about a virtual inclined line tilted with respect to the vertical line, with a plurality of solid medicine pieces being placed on an upper surface portion of the inner inclined rotary body, to move the plurality of medicine pieces onto the annular upper end surface of the outer rotary body while rotating; and
an alignment regulation mechanism configured to align the plurality of medicine pieces, which have been moved onto the annular upper end surface of the outer rotary body, in a rotational direction of the annular upper end surface when the outer rotary body is rotating, wherein:
the alignment regulation mechanism includes a tablet height regulation mechanism including a plurality of suspended objects suspended over the annular upper end surface, the tablet height regulation mechanism being configured to regulate heights of the medicine pieces using the plurality of suspended objects; and
the plurality of suspended objects include at least one deformable suspended object that is deformed when a lower end portion of the deformable suspended object is pushed sideways and at least one non-deformable suspended object that is not deformed when a lower end portion of the non-deformable suspended object is pushed sideways with an upper end portion of the non-deformable suspended object being swingably supported.

3. The medicine feeder according to claim 1 or 2, wherein
the at least one deformable suspended object and the at least one non-deformable suspended object are provided at different positions as seen in a circumferential direction of the outer rotary body.

4. The medicine feeder according to claim 1 or 2, wherein
the at least one deformable suspended object and the at least one non-deformable suspended object are provided at different positions as seen in a circumferential direction and a radial direction of the outer rotary body.

5. The medicine feeder according to claim 3, wherein
the plurality of suspended objects are arranged in the circumferential direction in order of the at least one deformable suspended object, the at least one non-deformable suspended object, and at least one different deformable suspended object.

6. The medicine feeder according to claim 1 or 2, wherein:
the at least one deformable suspended object is constituted to allow the lower end portion of the deformable suspended object to be deformed along an outer peripheral surface of the tablets and generate a repulsion force to disentangle the plurality of stacked tablets when a plurality of stacked tablets located on the annular upper end surface abut against the at least one deformable suspended object; and
the at least one non-deformable suspended object is constituted to generate a repulsion force to disentangle the plurality of stacked tablets and move the tablets toward the opening portion of the outer rotary body along the lower end portion of the non-deformable suspended object when a plurality of stacked tablets located on the annular upper end surface abut against the at least one non-deformable suspended object.

7. The medicine feeder according to claim 1 or 2, wherein:
the tablet height regulation mechanism includes at least one support arm configured to support respective upper end portions of the plurality of suspended objects;
a through hole penetrating in the vertical direction is formed in the support arm to be penetrated by the upper end portion of the at least one non-deformable suspended object;
a projecting portion projecting in the same direction as the upper end portion is provided between the upper end portion and the lower end portion of the at least one non-deformable suspended object; and
a slit portion is provided under the through hole, with the projecting portion being loosely fitted in the slit portion, to restrict motion of the projecting portion such that the at least one non-deformable suspended object is swung in a limited range when the stacked tablets abut against the lower end portion.

8. The medicine feeder according to claim 7, wherein:
an entire portion of the at least one non-deformable suspended object or a portion of the non-deformable suspended object including the lower end portion is in a plate shape;
the lower end portion includes a first portion located in a first virtual plane together with the projecting portion and a second portion that is continuous with the first portion and located in a second virtual plane intersecting the first virtual plane at a predetermined angle θ; and
the slit portion positions the at least one non-deformable suspended object such that the first portion extends along a rotational direction of the outer rotary body and the second portion is located on a rear side in the rotational direction with respect to the first portion and extends outward in a radial direction of the outer rotary body.

9. The medicine feeder according to claim 1 or 2, wherein
the at least one non-deformable suspended object is composed of a plurality of separable members arranged side by side.

10. The medicine feeder according to claim 1 or 2, wherein
the at least one non-deformable suspended object is composed of a plurality of separable plate materials of the same shape arranged side by side.

11. The medicine feeder according to claim 1 or 2, further comprising:
a control portion configured to control rotation of the outer rotary body; and
fallen medicine detecting means for detecting a fallen medicine piece that has been carried to a fall-discharge port by the outer rotary body, wherein:
the alignment regulation mechanism includes a lateral width regulation mechanism configured to regulate a lateral width of a medicine transfer path on the annular upper end surface of the outer rotary body;
the lateral width regulation mechanism is configured to adjust a regulation amount of the lateral width according to a control instruction from the control portion; and
the lateral width regulation mechanism increases the lateral width according to the control instruction from the control portion when an interval of detection for fallen medicine pieces by the fallen medicine detecting means reaches a congestion-time interval which is longer than a normal-time interval.

12. The medicine feeder according to claim 11, wherein
when the fallen medicine detecting means detects a fallen medicine piece after the lateral width regulation mechanism increases the lateral width according to the control instruction from the control portion, the lateral width regulation mechanism restores the lateral width before being increased according to a control instruction from the control portion provided according to such detection of the fallen medicine piece.

13. The medicine feeder according to claim 11 or 12, wherein
the lateral width regulation mechanism increases the lateral width stepwise according to the control instruction from the control portion.

14. The medicine feeder according to claim 11, further comprising:
a dimension measurement mechanism operable to clamp a medicine piece and measure a dimension of the medicine piece; and
a dimension measurement drive mechanism configured to move a movable portion of the dimension measurement mechanism, wherein:
the lateral width regulation mechanism is configured to set an initial value of the lateral width in conjunction with the movable portion of the dimension measurement mechanism; and
when the control portion acquires medicine dimension data on the medicine piece placed on a dimension measurement portion of the dimension measurement mechanism by driving the dimension measurement drive mechanism, the control portion outputs the control instruction to cause the lateral width regulation mechanism to regulate the lateral width based on the acquired medicine dimension data.

15. The medicine feeder according to claim 1 or 2, wherein:
the tablet height regulation mechanism includes an elevating mechanism operable to elevate and lower the plurality of suspended objects;
the medicine feeder further includes first data storage means for holding the medicine dimension data acquired by the dimension measurement mechanism, and second data storage means for acquiring and holding medicine dimension data transmitted from an upper-level device; and
the lateral width regulation mechanism is caused to regulate the lateral width and the elevating mechanism of the tablet height regulation mechanism is caused to regulate the height based on the medicine dimension data held by one of the first data storage means and the second data storage means.

16. The medicine feeder according to claim 15, wherein
the first data storage means and the second data storage means share a data storage memory.

17. The medicine feeder according to claim 15, wherein
when the control portion acquires the medicine dimension data from the first data storage means, the control portion transmits the medicine dimension data to the upper-level device.

18. A medicine dispensing system comprising:
a medicine dispensing apparatus in which the medicine feeder according to claim 17 is mounted in a medicine storage; and
a medicine dispensing server configured to hold prescription data based on information on a prescription and a medicine master including a collection of data on various types of medicine, wherein:
the medicine dispensing server and the medicine dispensing apparatus are able to transmit and receive data, and a control portion of the medicine dispensing apparatus and the control portion of the medicine feeder are able to transmit and receive data; and
when the medicine dispensing apparatus receives medicine dispensation instruction data including medicine to be processed by the medicine feeder from the medicine dispensing server, the medicine dispensing apparatus checks whether or not the medicine dispensation instruction data include medicine dimension data on the medicine, and transmits the medicine dimension data to the medicine feeder if the medicine dimension data are included, and instructs the medicine feeder to acquire medicine dimension data using the dimension measurement mechanism and transfers the medicine dimension data transmitted from the medicine feeder to the medicine dispensing server if not.
